Europäisches Patentamt

European Patent Office

Office européen des brevets

(19)

(11) Publication number: **0 344 319**

**A1**

(12)

# EUROPEAN PATENT APPLICATION
## published in accordance with Art. 158(3) EPC

(21) Application number: 88908977.7

(22) Date of filing: 14.10.88

(86) International application number:
PCT/JP88/01044

(87) International publication number:
WO 89/03425 (20.04.89 89/09)

(51) Int. Cl.⁴: **C12N 15/00 , C12P 21/00**

(30) Priority: 14.10.87 JP 259206/87
09.07.88 JP 170041/88

(43) Date of publication of application:
06.12.89 Bulletin 89/49

(84) Designated Contracting States:
**BE CH DE FR GB IT LI NL SE**

(71) Applicant: **MITSUI TOATSU CHEMICALS, Inc.**
**2-5 Kasumigaseki 3-chome**
**Chiyoda-Ku Tokyo 100(JP)**

Applicant: **MITSUI PHARMACEUTICALS, INC.**
**12-2, Nihonbashi 3-chome Chuo-ku**
**Tokyo 103(JP)**

Applicant: **EDUCATIONAL FOUNDATION**
**FUJITA GAKUEN**
**12-1, Minamiyakata, Sakaemachi**
**Toyoake-shi, Aichi 470-11(JP)**

(72) Inventor: **KUROSAWA, Yoshikazu 2-215,**
**Raionzu Manshon**
**Yagoto Gaaden 20-1, Yagotofujimigaoka**
**Tenpakucho**
**Shouwa-ku, Nagoya-shi, Aichi 466(JP)**
Inventor: **AWAYA, Akira 2-3, Daini Apaato**
**1541, Yabecho Totsuka-ku**
**Yokohama-shi Kanagawa 244(JP)**
Inventor: **ISHIZUKA, Yusaku**
**21, Honmokuosatocho Naka-ku**
**Yokohama-shi Kanagawa 231(JP)**

(74) Representative: **Holdcroft, James Gerald, Dr.**
**et al**
**Graham Watt & Co. Riverhead**
**Sevenoaks Kent TN13 2BN(GB)**

(54) IMMUNOGLOBULIN GENE-RELATED DNA FRAGMENT.

(57) Specific DNA sequences such as $\sigma\mu$, $\sigma\gamma 3$, $\sigma\gamma 1$, $\sigma\gamma 2$ and $\sigma\gamma 4$ different from switch sequences conventionally known in subclass production of antibody are cloned, and the DNA sequences themselves and DNA sequences homologous therewith are revealed from the obtained clones. It is clarified by using the clones that $\Sigma\mu$ gene having homology with $\sigma\mu$ and $\Sigma\gamma$ gene having homology with $\sigma\gamma$ exist adjacent to each other in the region between $C\mu$ and $C\delta$ ($\Sigma$ region), and their functions in class switch are being demonstrated. The clones of the present invention enables specific control of production of a subclass of antibody against various type and kind of antigen.

# DNA FRAGMENTS RELATED TO IMMUNOGLOBULIN GENE

## TECHNICAL FIELD

The present invention concerns new DNA fragments which relate to a class switch mechanism in immunoglobulin production in mammalians, particularly in higher animals, in the fields of immunology, genetics and cancer research. Furthermore, the present invention concerns expression itself of the DNA fragments and a method of producing class specific immunoglobulins using functions of these DNA fragments or a technology necessary to establish cell lines to be used in this method.

## BACKGROUND ART

Owing to the research development immunological genetics, it has been proved that immunoglobulin genes are those to be expressed specifically in B and B-related cells and that the expression is controlled according to the process of differentiation and maturation of B cells (see Tonegawa, S. Molecular biology in immunological recognition. Protein, nucleic acid, enzyme, $\underline{32}$ (3), 239, 1987 and Kurosawa, Y. Origin of diversity for immune system, $\underline{31}$ (9), 756, 1986).

The total organization of human immunoglobulin heavy chain (hereinafter referred to as H chain) genes in order is as follows: H chain variable region (hereinafter referred to as $V_H$) genes, H chain D region (hereinafter referred to as $D_H$) genes, H chain J region (hereinafter referred to as $J_H$) genes and constant region (hereinafter referred to as C) genes.

The order of the constant genes is $C_\mu$-$C_\delta$-$C_\gamma3$-$C_\gamma1$-$C_{\psi e}$-$C_\alpha1$-$C_\gamma2$-$C_\gamma4$-$C_e$-$C_\alpha2$. Characteristic repetitive sequences referred to as switch sequences (hereinafter referred to as S) exist upstream of each C gene except for the $C_0$ gene. Namely they are $S_\mu$, $S_\gamma3$, $S_\gamma1$, $S_{\psi\epsilon}$, $S_\alpha1$, $S_\gamma2$, $S_\gamma4$, $S_\epsilon$ and $S_\alpha2$.

During B cell ontogeny, these genes undergo two kinds of DNA rearrangements. One is a $V_H$-$D_H$-$J_H$ joining, resulting in the formation of a completely active type V gene and the other is recombination which undergoes between two switch sequences (e.g., between $S_\mu$ and $S_\gamma$) for the H chain class switch.

Both rearrangements usually involve the loss of the intervening sequences. Furthermore, the enhancer sequence has been identified just downstream of the $J_H$ genes. Outline of the knowledge so far elucidated is as described above, and thus, lots have been remained unknown regarding control mechanisms of immunoglobulin production.

## DISCLOSURE OF THE INVENTION

An object of the present invention is to provide extremely useful technology for elucidation of regulatory mechanisms of production of immunoglobulins and its applications in medicinal or industrial field.

The present inventors have attempted to elucidate unsolved problems regarding the mechanism, in which B cell family undergoes ontogeny responding to various antigens, interactions occur between the antibody genes during the ontogeny, and thus various antibodies are produced, as well as the mechanism of class specific antibody production. In the course of such attempts, the present inventors assumed the presence of an independent unknown switch mechanism in class switch of immunoglobulins at the level of DNA or RNA, which regulates functions at the level of various genes especially in H chains. Furthermore, the present inventors considered that the mechanisms to elucidate such phenomena and facts must be revealed, if, in human antibody genes, unidentified DNA nucleotide sequences in new $D_H$ gene families, new $J_H$ gene families or $J_H$-enhancer-$S_\mu$-$C_\mu$-$C_\delta$ gene sequences especially in intron regions and DNA nucleotide sequences of the intron regions near the S gene just upstream of individual $C_\gamma$ genes downstream of $C_\gamma3$ are investigated in detail.

Accordingly, the present inventors have made great efforts to determine the aforementioned genes and DNA nucleotide sequences and revealed that a copy (having homology; named $\Sigma\mu$ gene) of DNA segment ($\sigma_\mu$) of intron, which locates between the enhancer and switch sequence $S_\mu$ upstream of $C_\mu$ of the human immunoglobulin heavy chain gene residing on chromosome 14, is present (being inserted) in another region of the germline chromosome 14, namely, in the upstream region of $C_\delta$ downstream of $C_\mu$, and further, that

the DNA segment (hereinafter referred to as $\Sigma_x$) homologous to the gene ($\sigma_x$) located upstream of the individual constant region genes exists in groups near the aforementioned DNA segment ($\Sigma_\mu$).

The present inventors referred to this complex region of the unexpected DNA segments as Sigma ($\Sigma$) region, revealed that the gene complex containing $\Sigma_x$ and $\Sigma_\mu$ was a new gene complex which controls class switch mechanisms of immunoglobulin genes by comparing it with mouse immunoglobulin H chain genes simultaneously analyzed, established cells capable of producing class specific immunoglobulins by expression or loss of the genes in this gene complex region, and then completed techniques necessary for a method to produce class specific immunoglobulins.

Furthermore, the present inventors assigned two indices for biologically significant regions in introns, and compared human and mouse gene nucleotide sequences in detail. Namely, one aspect is homology the genetically same regions in human and mouse genes, in other words, the presence of nucleotide sequences which are common to mouse and human genomes and conserved in human genome during the process of evolution, and the other aspect is the presence of nucleotide sequences which behave like transposons. By using this methodology, the present inventors carried out identification of the DNA sequences in and around the aforementioned Sigma region, in and around $\sigma_\mu$ region and in and around $\sigma_x$ region further upstream of the class switch region ($S_x$) upstream of individual constant region genes, characterized $\sigma_\mu$, $\sigma_x$, BamHI-HindIII fragments in mouse $\gamma_{2b}$-$\gamma_{2a}$ intron, sigma gamma core fragment and sigma delta core fragment, as fragments to satisfy the abovementioned two indices, and further succeeded in establishing techniques necessary to elucidate a mechanism of alternative splicing from a primary transcript (RNA) (Yaoita, Y., Y. Kumagai, K. Okumura and T. Honjo: Expression of lymphocyte surface IgE does not require switch recombination. Nature, 297, 697-699, 1982) in B cells which simultaneously express two different isotypes such as a combination of IgM and IgG and techniques necessary to elucidate relevant DNA sequences. In the course of the investigation, the present inventors identified promoter activity regions for transcription by RNA polymerase III upstream of $C_\gamma$ genes, which can be involved in the discontinuous transcription followed by trans-RNA splicing in which constant region $C_\gamma$ gene's transcription takes place independently from ordinary one initiated from promoter regions of $V_H$-$D_H$-$J_H$ genes.

Furthermore, the present inventors revealed that IgD is relatively abundantly produced specifically in human immunoglobulins because of the presence of DNA rearrangements which occur between $\sigma_\mu$ and $\Sigma_\mu$.

According to the present invention, besides the switch sequences conventionally known in production of antibody subclasses, specific DNA fragment sequences such as $\sigma_\mu$, $\sigma_\gamma 3$, $\sigma_\gamma 1$, $\sigma_\gamma 2$ and $\sigma_\gamma 4$ were cloned to identify their nucleotide sequences homologous to each other.

Furthermore, it was revealed that $\Sigma_\mu$ having homology to part of $\sigma_\mu$ and $\Sigma_\gamma$ gene having homology to part of $\sigma_\gamma$ are located neighboring each other in the region between $C_\mu$ and $C_\delta$ (Sigma region), which may partly demonstrate their functions in the class switch.

On the basis of the fact demonstrated according to the present invention and by use of relevant techniques, it is possible to specifically control production of subclasses of antibodies against various types and kinds of antigens.

Namely, in production of monoclonal antibodies against specific antigens, IgG type antibodies are often desired rather than IgM type antibodies and accordingly it is expected that the mechanism of antibody subclass production, which is elucidated according to the present invention, may be applied as the principle in controlling the antibody production system so as to predominantly produce IgG type antibodies. Thus, a variety of methodology to prepare differentiated antibody producing cells can be developed.

Furthermore, IgD producing cells are conventionally double producing cells which produce IgM and IgD, but the cells which produce only IgD are also known. In IgD producing cell lines, DNA rearrangement may possibly occur with sdc sequence-containing $\sigma_\mu$ and $\Sigma_\mu$ as signals.

The significance and roles of the cells which produce only IgD are elucidated on the basis of information and techniques obtained according to the present invention.

On the other hand, sgc sequence upstream of $C_\gamma$ gene, identified by the present inventors, and sdc sequence in $C_\mu$-$C_\delta$ intron are found in both mouse and human, and their primary transcripts (RNAs) can form tRNA-like secondary structures. Sequences capable of constructing the primary transcripts complementary to these sdc sequence and sgc sequence are present in $J_H$-$\mu$ intron and $C_\mu$-$C_\delta$ intron, respectively, and each of these sequences can play an important role in RNA splicing in $\mu$-$\delta$ (IgM and IgD) double producing cells or $\mu$-$\gamma$ (IgM and IgG) double producing cells.

The $\mu$-$\gamma$ double producing cells are changed in the process of cell differentiation into IgM or IgG producing cells. On the process of RNA splicing, IgG can be selectively produced using a mechanism in which genes coding for IgG rather than IgM are dominantly formed.

Thus according to the present invention, cloning of the such differentiated cells that produce IgG only is motivated, and substantially, it is expected that such a cell group can be inevitably prepared if cells producing specifically useful IgG antibodies are thoroughly searched.

On the other hand, RNA polymerase III promoter regions identified upstream of sgc sequences in $\sigma_\gamma 3$ and $\sigma_\gamma 4$ of immunoglobulin genes whose activity was evidenced in vitro are considered to work practically proceeding RNA trans-splicing in vivo. Subsequently, using the RNA splicing mediated with this promoter activity and further with the sgc sequence and sdc sequence and the like, the compositions of proteins produced in production of various proteins using genetic engineering technology can be changed as desired, which realizes production of various types and kinds of proteins.

Namely, it is extremely useful to develop a new method for producing known proteins or to produce new protein mutants using various fragments such as sdc or sgc sequences having capability of effectively trans-splicing RNAs which have been independently transcribed with several independent promoters.


BRIEF DESCRIPTION OF DRAWINGS


Figures 1 (a), (b) and (c) are restriction enzyme maps of the immunoglobulin heavy chain constant gene loci and the clones (thick lines: ARAJH1, clones 1, 2, 3 and 6) isolated according to the present invention. In the drawings, cleavage sites for the restriction enzymes HindIII, EcoRI and BamHI are shown by ▼ , ▽ and↓, respectively. Furthermore, the sites where the nucleotide sequences were determined in the present invention are shown by ↔.

Figure 2 shows nucleotide sequences of $\Sigma_\mu$ in the Sigma ($\Sigma$) region and $\sigma_\mu$ in the $J_H$-$C_\mu$ intron, in which sequences homologous to each other are indicated. In this drawing, boundaries between homologous and non-homologous sequences are shown by vertical lines, and further nucleotides that are different in the homologous regions are shown by ● marks.

Figure 3 is a drawing to show parindoromic property in the human $\sigma_\mu$ regions. Arrows indicate the boundaries between the regions homologous and non-homologous to each other between the $J_H$-$C_\mu$ intron and $C_\mu$-$C_\delta$ intron, and the sequences of the upstream and downstream ends of $C_\mu$-$C_\delta$ intron are shown on the right hand up and the left hand up, respectively. Further, the sites complementary to sdc sequence are shown in boxes.

Figure 4 is a graphical drawing showing DNA rearrangement by the interaction of $\Sigma_\mu$ and $\sigma_\mu$.

Figure 5(A) shows an outline of H chain genes, Figure 5(B) is a graphical drawing to demonstrate switching at DNA level by the action of $\Sigma_\mu$ and $\sigma_\mu$, and Figure 5(C) is a graphical drawing to demonstrate switching at RNA level by the action of $\Sigma_x$ and $\sigma_\gamma 3$.

Figure 6 shows repeat sequences related to $\sigma_\gamma 1$, $\sigma_\gamma 2$, $\sigma_\gamma 3$ and $\sigma_\gamma 4$.

Figure 7 is a graphical drawing to demonstrate rearrangement at DNA level by the action of $\Sigma_x$ and $\sigma_\gamma 3$. $R_\gamma 3$ shows a repeat sequence.

Figure 8 shows the result of Southern hybridization of DNAs of the following haematopoietic cell lines:

Lane A: Human placenta germline cell
Lane B: HLA60 cell
Lane C: ARA10 cell
Lane D: Jurkat cell
Lane E: Daudi cell


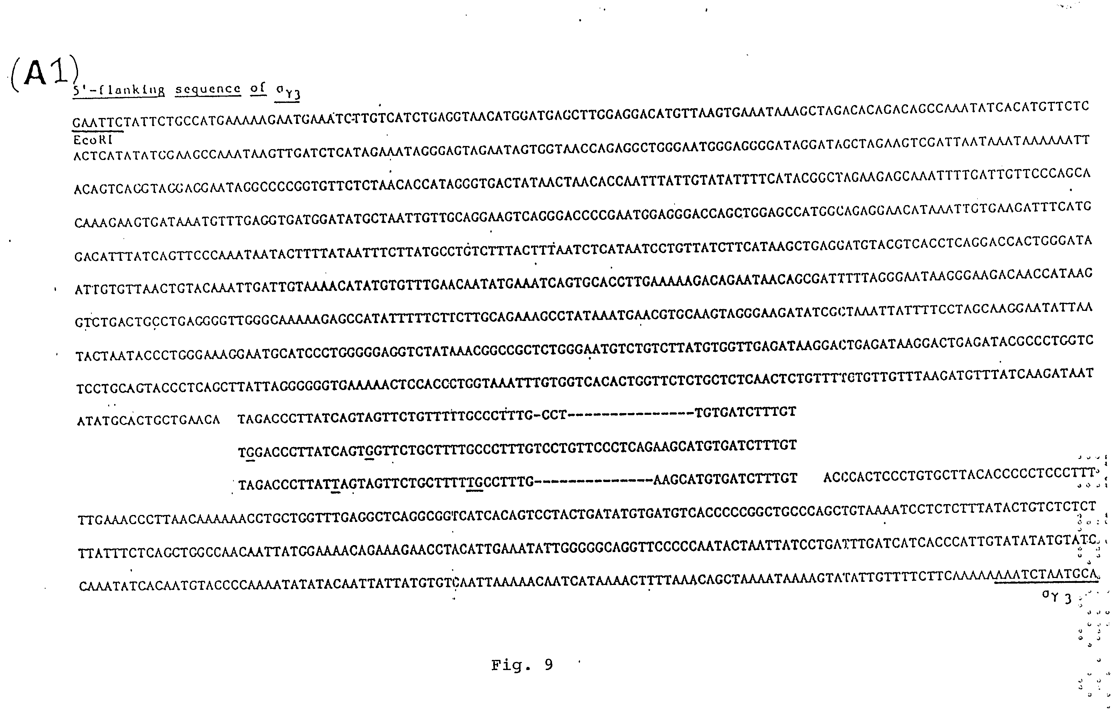

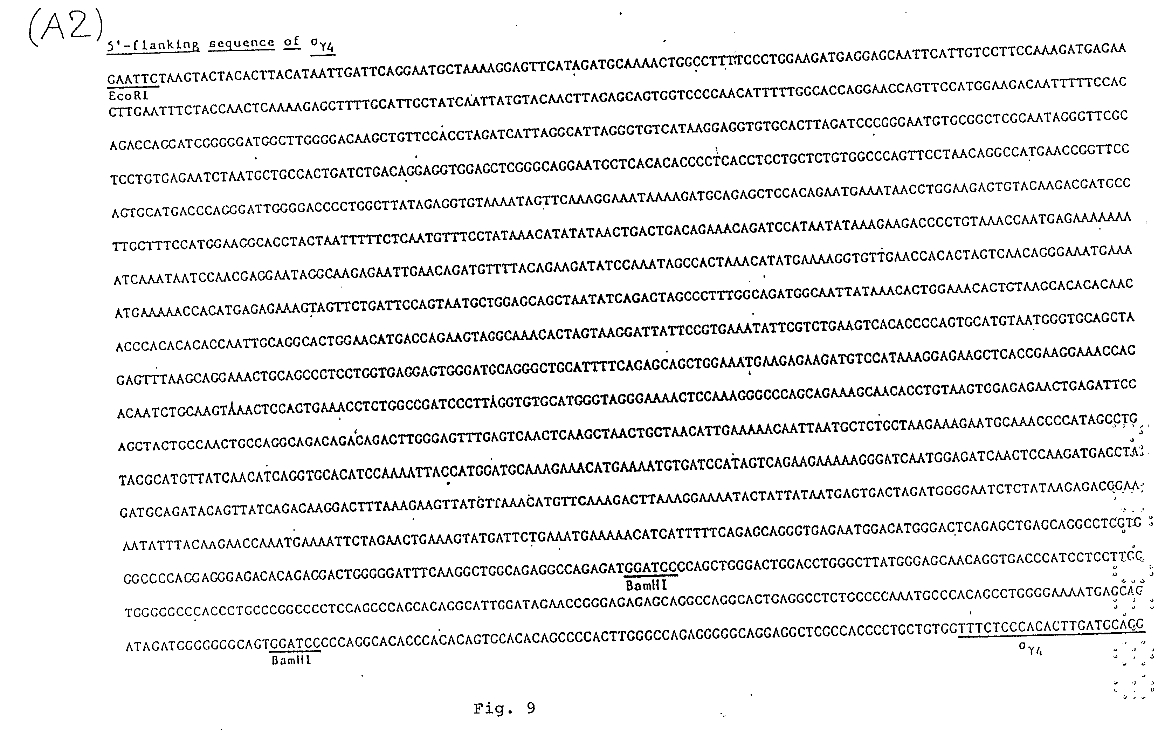

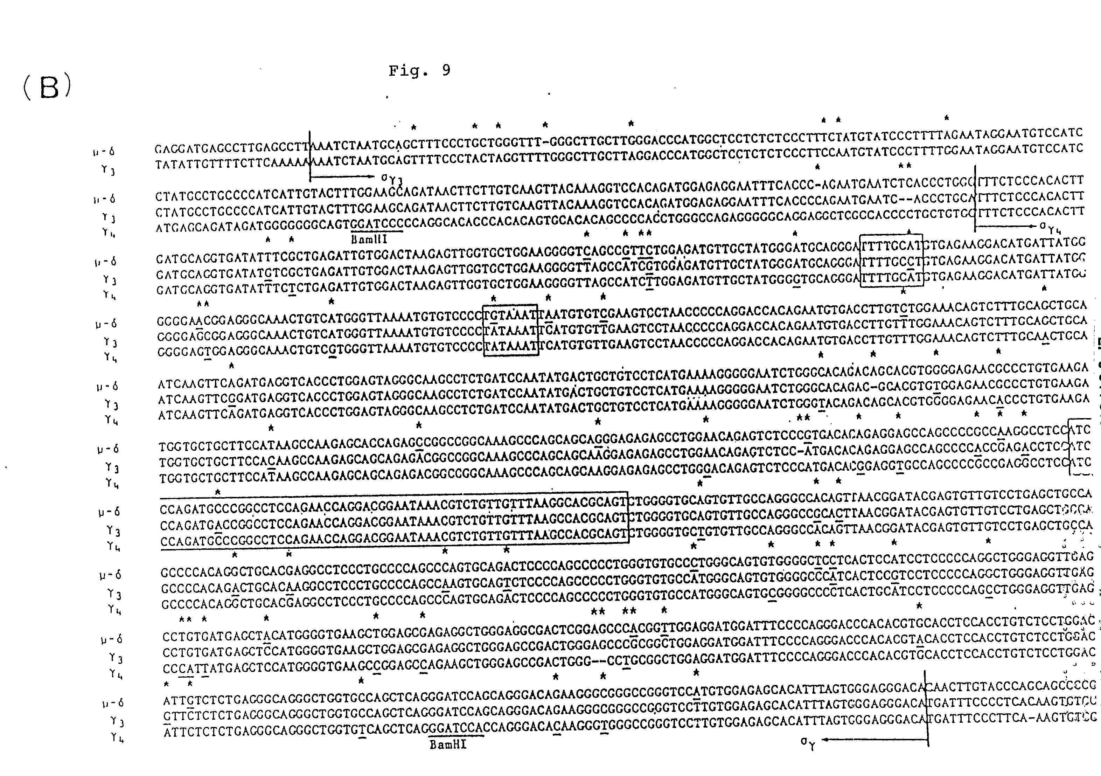

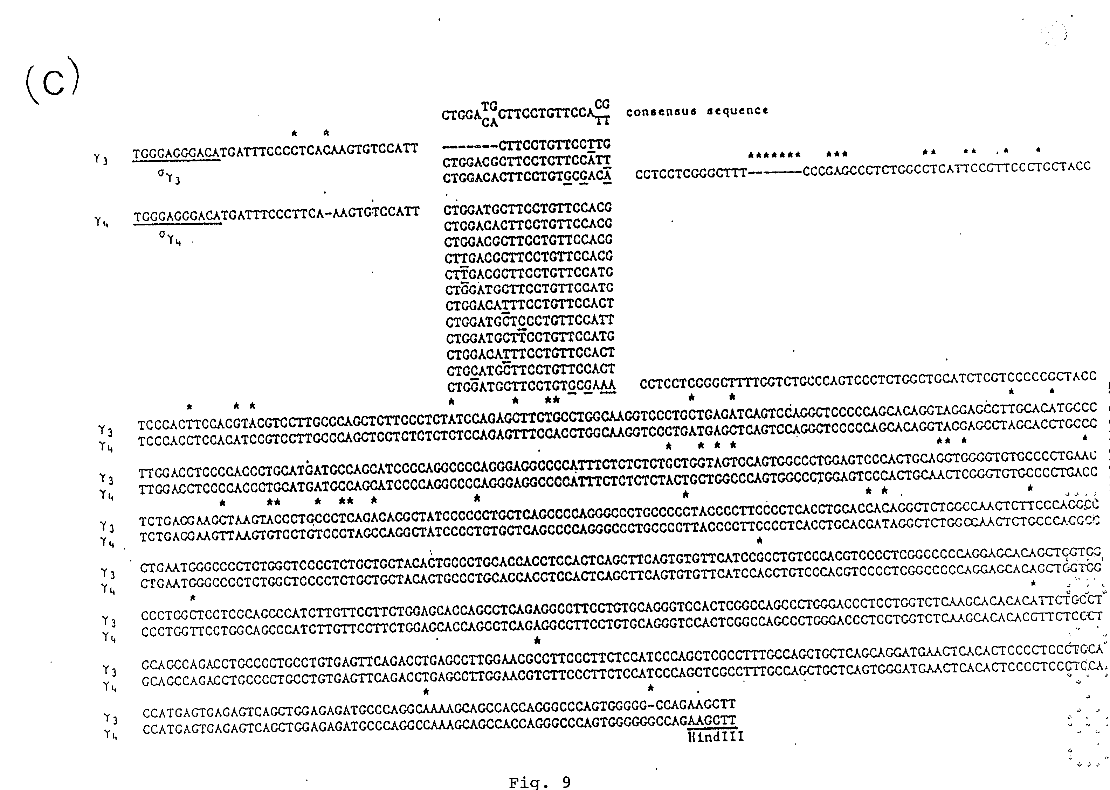

Figures 9(A1) - (C) are shown to demonstrate comparison of nucleotide sequences of one moiety of nucleotide chains of EcoRI-HindIII fragment containing $\sigma_\gamma 3$ and EcoRI-HindIII fragment containing $\sigma_\gamma 4$, both identified in the present invention, with a known nucleotide sequence between $C_\mu$-$C_\delta$ intron. Figure 9(A1) shows a nucleotide sequence of the 5′ side upstream of $\sigma_\gamma 3$ region of the EcoRI-HindIII fragment containing $\sigma_\gamma 3$, Figure 9(A2) shows a nucleotide sequence of the 5′ side upstream of $\sigma_\gamma 4$ region of the EcoRI-HindIII fragment containing $\sigma_\gamma 4$, Figure 9(B) shows comparison of nucleotide sequences of $\sigma_\gamma 3$, $\sigma_\gamma 4$ and $C_\mu$-$C_\delta$ intron ($C_\mu$-$C_\delta$ intron is shown in the opposite direction from the 3′ end), and Figure 9(C) shows comparison of nucleotide sequences downstream of $\sigma_\gamma 3$ and $\sigma_\gamma 4$. In these drawings, nucleotides that are different are marked by asterisks and underlined. Further, bars indicate the absence of corresponding nucleotides. Boundaries between homologous and non-homologous sequences are shown by vertical lines, and TATA-like sequences, octamer-like sequences required for expression of Ig genes and sgc sequence are boxed.

Figure 10(a) demonstrates orientations of repeat sequences found in the sequence between $C_\mu$-$C_\delta$ intron of the human and mouse genomes. Figure 10(b) demonstrates the relationships between the 63 bp repeat sequence (in Sigma region and downstream of $\Sigma_\mu$) in human $C_\mu$-$C_\delta$ intron and the sequences in the $\sigma_\gamma 3$, $\sigma_\gamma 4$ and mouse $C_\mu$-$C_\delta$, which have homology to part of these sequences.

Figures 11(a) and (b) independently show cloverleaf structures formed based on tRNA and sgc sequence.

Figure 12(a) shows a restriction enzyme map of MEP12 containing mouse $C_\gamma 2b$-$C_\gamma 2a$ intron, Figure 12(b) shows a nucleotide sequence of the BamHI-HindIII fragment containing the boxed sgc sequence, and Figure 12(c) shows a tRNA-like secondary structure which can be formed based on the sgc sequence in Figure 12(b).

Figure 13(a) shows a secondary structure from the mouse sdc sequence, Figure 13(b) shows a secondary structure from the human sdc sequence, Figure 13(c) shows a hybridization structure in the primary transcript from sdc sequence and $\sigma_\mu$ in mouse, figure 13(d) shows a hybridization structure in the primary transcript from sdc sequence and $\sigma_\mu$ in human, and Figure 13(e) shows a secondary structure of the primary transcript from regions containing mouse $\sigma_\mu$. The sequences complementary to the mouse sdc are boxed.

Figures 14(A) and (B) show hybridization structures each of which can be formed in the primary transcript corresponding to the sites from $V_H D_H J_H$ to $C_\gamma$ gene in the complementary region.

Figures 15(A) and 15(B) show structures of pSG3 with cloned $\sigma_\gamma 3$ and pSG4 with cloned $\sigma_\gamma 4$, respectively. Cleavage sites for EcoRI, BamHI and HindIII are indicated by E, B and H, respectively, and sgc sequences are indicated by ■ marks.

## BEST MODE FOR CARRYING OUT THE INVENTION

A recombinant DNA according to the present invention containing individual genes described hereinafter in detail is useful as a material in utilizing functions of DNA fragments of these genes for various purposes and as an agent to be used in order to confirm new findings by the inventors as explained above or to further elucidate mechanisms to control immunoglobulin production.

Representative examples of the recombinant DNA according to the present invention are clones 1, 2, 3 and 6 and clones containing ARAJH1, pSG 3, pSG 4 or $\sigma_\gamma 1$ gene and a clone containing $\sigma_\gamma 2$ gene.

A recombinant DNA according to the present invention can be prepared by a method comprising treating, for example, human immunoglobulin heavy chain genes with appropriate restriction enzymes to make a fragment, binding this fragment to an appropriate vector, constructing a DNA library and then selecting clones containing desired genes from the library.

For construction of the DNA library, vectors comprising various plasmids and phages extensively used for cloning can be used.

An example in which a vector derived from lambda phage is used as a vector will be described below.

First, an appropriate haematopoietic cell DNA containing human immunoglobulin heavy chain DNA genes is prepared according to the ordinary method and then treated with appropriate restriction enzymes, and the resultant DNA fragment is bound to a vector derived from lambda phage such as Charon 4A vector so as to construct a DNA phage library.

Separately, an appropriate haematopoietic cell DNA containing human immunoglobulin heavy chain genes is prepared by the ordinary method and then treated with the restriction enzyme HindIII. From the resultant cleaved fragment mixture, using DNA containing heavy chain $J_H$ gene (containing a known nucleotide sequence) as a probe, hybridization was carried out according to an ordinary method for screening, and a 3.8 kb DNA fragment is isolated. As a clone containing this 3.8 kb DNA fragment, ARAJH1 was isolated in Examples described hereinafter.

Subsequently, from the DNA phage library obtained above, a recombinant which hybridizes with this 3.8 kb DNA is selected according to the plaque hybridization method or the like.

Whether the selected clones contain desired genes can be confirmed by constructing restriction maps or analyzing DNA sequences.

Further, an example of the aforementioned DNA phage library to be used is Maniatis' human DNA phage library (Lawn, R. M., E. F. Fritsh, R. C. Parker, G. Blake and T. Maniatis: The isolation and characterization of linked delta- and beta- globulin genes from a cloned library of human DNA. Cell, 15, 1157-1174, 1978).

Further, the screening with the use of ARAJH1 is carried out, for example, by the Benton-Davis method (Benton, W. D. and R. W. Davis: Screening lambda-gt recombinant clones by hybridization to single plaque in situ. Science, 196, 180-182, 1977).

Further, clones with insertion of desired genes into vectors derived from lambda phage are grown in suitable bacterial cells as a host so as to make them applicable for various usages.

As in the case where Maniatis' human DNA phage library is used, when a lambda phage vector such as Charon 4A vector is used, supE$^+$ Escherichia coli, e.g., E. coli DP 50, E. coli 803 [for example, provided by K. Murray (University of Edinburgh)], E. coli K-12 x 1776 (ATCC 31244;), E. coli K 802 (ATCC 33526), E. coli LE 392 (ATCC 33572), E. coli MM 29d (ATCC 33625) or E. coli MM21 (ATCC 33678) can be used as a host.

The following descriptions serve to illustrate the individual genes, grouped according to their major functions, cloned in the recombinant DNA according to the present invention described above.

These functions of the genes were newly found by the present inventors.

1. Genes involved in DNA level rearrangement

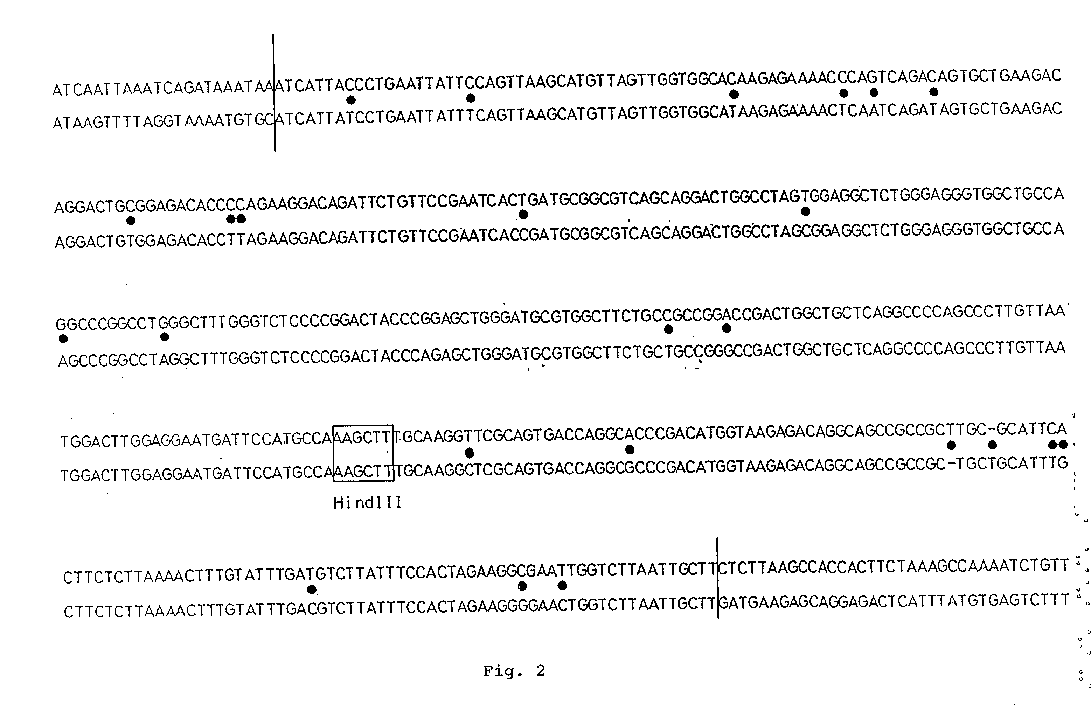

The $\Sigma_\mu$ gene in the present invention is defined as a gene consisting of a DNA sequence which has homology to part of $\sigma_\mu$ gene [shown in Fig. 2 (in the lower row, between vertical lines)] located in the intron site present between an enhancer and a switch sequence $S_\mu$ upstream of $C_\mu$ in human immunoglobulin heavy chain genes and further having a nucleotide sequence shown in Fig. 2 (in the upper row, between vertical lines) found in the upstream region of $C\delta$ gene (Sigma region).

Furthermore, the $\Sigma_x$ gene is defined as a gene, which has homology to part of $\sigma_x$ gene (wherein x is one or more selected from the group consisting of $\gamma_3$, $\gamma_1$, $\psi_\epsilon$, $\alpha_1$, $\gamma_2$, $\gamma_4$, $\epsilon$ and $\alpha_2$) and found in the upstream region of $C\delta$ gene (Sigma region). Further, $\Sigma_x$ ($\Sigma_\gamma 1$-$\gamma 4$) which has homology to part of any of $\sigma_\gamma 3$, $\sigma_\gamma 1$, $\sigma_\gamma 2$ and $\sigma_\gamma 4$ is hereinafter optionally referred to as $\Sigma_\gamma$.

Furthermore, the Sigma region used in the present invention is defined as a region containing a complex region consisting of $\Sigma_\mu$ gene and $\Sigma_x$ gene, which is found between $C\delta$ gene and $C_\mu$ gene.

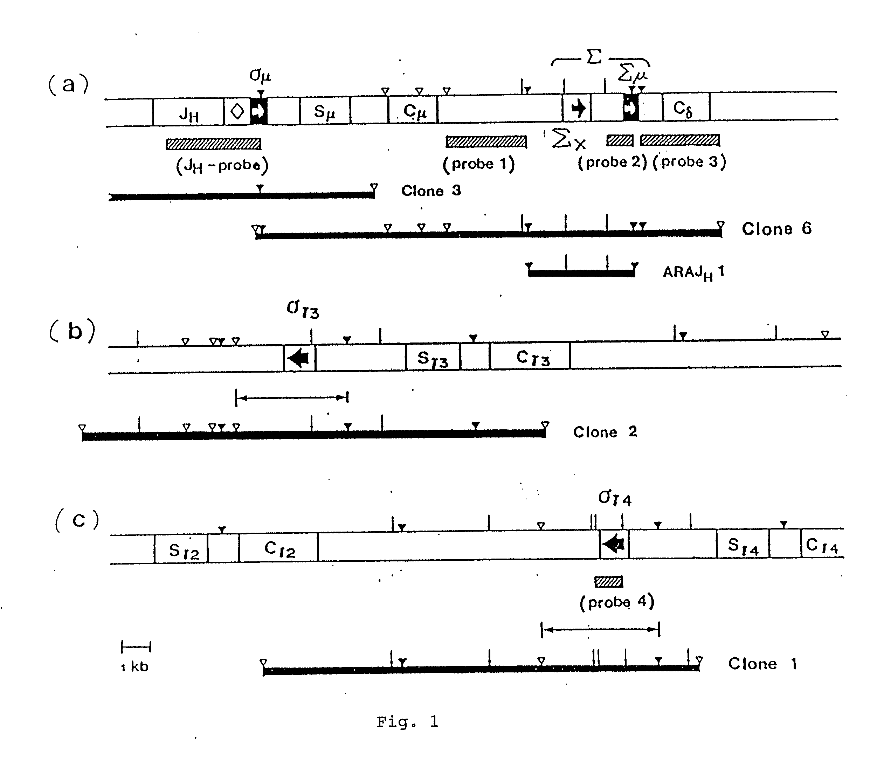

These genes are graphically shown in the physical maps of the H chain genes (germline chromosome 14) in Fig. 1.

Functions of the gene group relevant to the Sigma region, namely $\sigma_\mu$ gene, $\Sigma_\mu$ gene, $\Sigma_x$ gene and $\sigma_x$ gene (one or more genes selected from the group consisting of $\sigma_\gamma 3$, $\sigma_\gamma 1$, $\sigma_\psi e$, $\sigma_\alpha 1$, $\sigma_\gamma 2$, $\sigma_\gamma 4$, $\sigma_\epsilon$ or $\sigma_\alpha 2$) are considered to be in the following aspects.

The $o_\mu$ gene is located downstream of an enhancer and, when compared with the corresponding mouse DNA nucleotide sequence, homologous sequences were found in the Sigma region, which was pointed out previously but its meaning was not yet elucidated.

The present inventors investigated the details of the region containing this $\sigma_\mu$ gene and found a highly palindromic structure in the region (refer to the nucleotide sequence located in $J_H$-$C_H$ region, reported in Rabbitts et al.: Nucleic Acids Res., 12, 6523-6534, 1984).

In general, palindromic structures are often found in replication origins or between the respective ends of transposons.

On the other hand, for example, in switching of the constant region genes (except for the $C\delta$), repetitive sequences, referred to as switch sequences, are known and proved to be essential. Moreover, it is possible that binding sites for switching are not limited to the so-called switch sequences but rather widely distributed, but the sites are considered to be extended to the $\sigma_\mu$ and $\sigma_x$ genes specified by the present inventors.

In the upstream region of $C\delta$, the so-called switch sequences were not found, and it was newly proved that there exists Sigma region containing $\Sigma_\mu$ having homology to part of $\sigma_\mu$ gene.

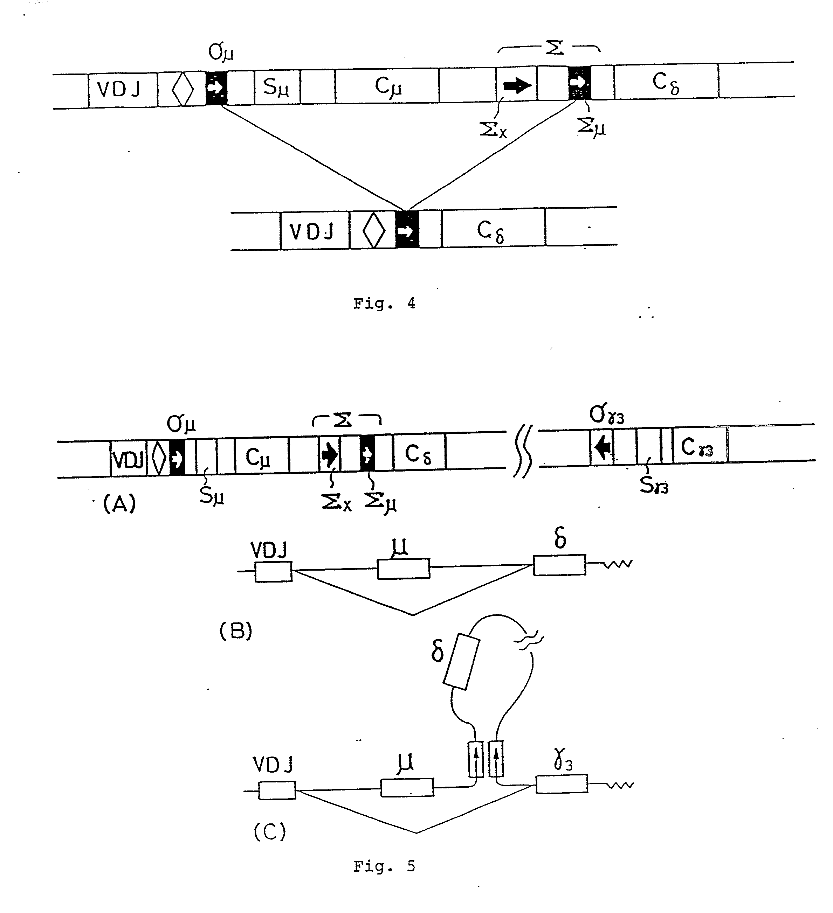

The abovementioned findings suggested that, aside from the class switch via so-called switch sequences presently known in the process of B cell ontogeny, the class switch in which DNA rearrangements occur at DNA level using $\sigma_\mu$ and $\Sigma_\mu$ genes as signals as shown in Fig. 4 may occur. Hence, the reason why the incidence of IgD is higher in human than in mouse, which have not so far been explained, could be explained by this mechanism.

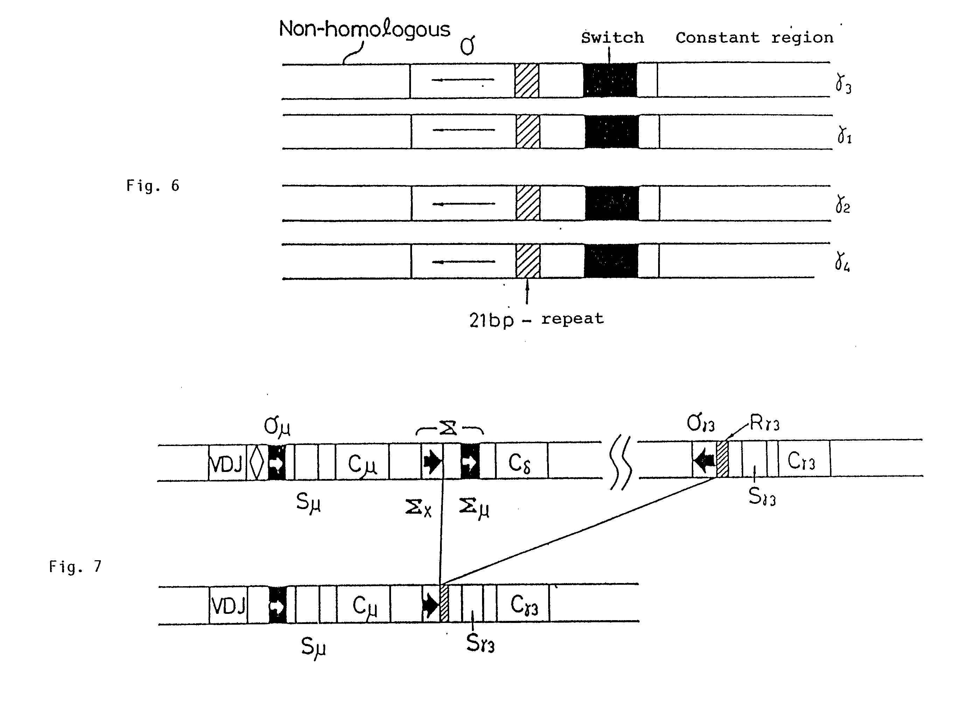

On the other hand, as to $\Sigma_\gamma$, $\sigma_\gamma 1$, $\sigma_\gamma 2$, $\sigma_\gamma 3$ and $\sigma_\gamma 4$, DNA rearrangements shown in Fig. 7 were found.

Furthermore, individual genes involved in the aforementioned DNA rearrangements can be cloned according to the method shown in Examples hereinafter described.

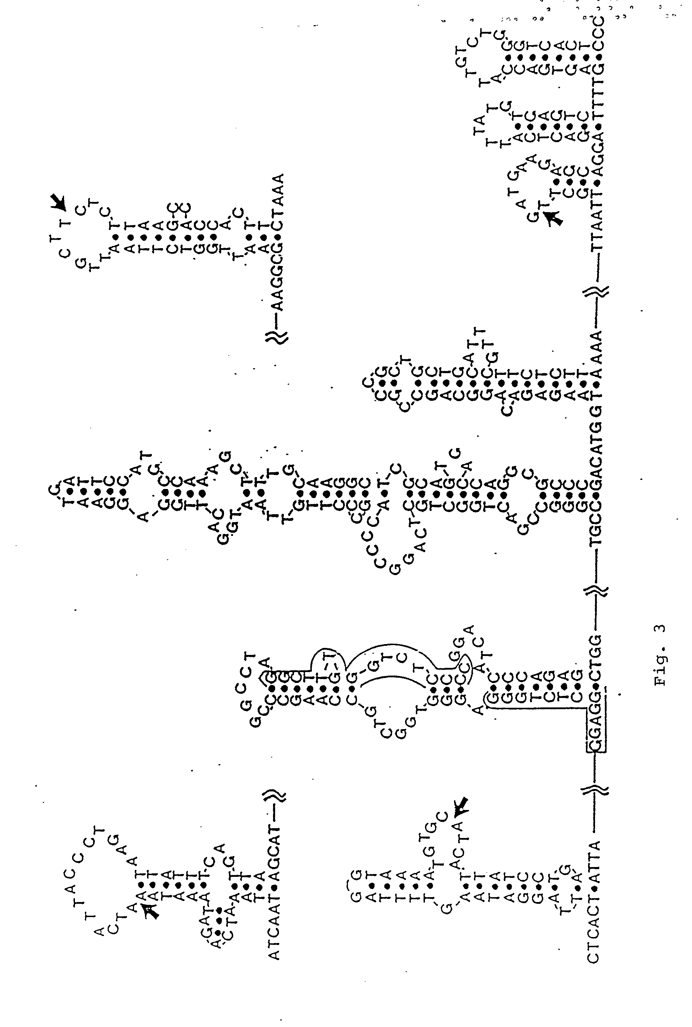

Furthermore, the $\sigma_\mu$ region homologous to part of the $\Sigma_\mu$ region and its neighboring region in human are highly palindromic as shown in Fig. 3, and such characteristics are known to be conserved in human and mouse systems. This may show that this $\sigma_\mu$ region and its neighboring regions are deeply involved in the aforementioned rearrangement at DNA level or in the splicing at RNA level as will be explained below.

## 2. Genes involved in splicing in mRNA

The aforementioned $\Sigma_x$ and $\sigma_x$ may be mainly involved in RNA splicing at mRNA level as shown in Fig 5(B).

Namely, as to $\mu$-$\delta$ double producing B cells in the production of immunoglobulins in B cells, the $\mu$-$\delta$ double producing B cells are dominating, and it seemed to be quite possible that there exists a strong relationship between such phenomena that B cells producing only $\delta$ are few or that the cells exclusively producing o are relatively abundant in human and the fact that there exists an analogous sequence (copy sequence; $\Sigma_\mu$) consisting of 442 nucleotides of $\sigma_\mu$ gene downstream of an enhancer upstream of $C_\delta$ [Fig. 5-(B)].

On the other hand, $\sigma_x$ gene which was newly found by the present inventors further upstream of a switch sequence located upstream of $C_x$ coding for the constant region can also possibly be involved in switching of the individual $C_x$, and is considered to play an important role, for example, in putative $\mu$-$\gamma$ double producing B cells besides the $\mu$-$\delta$ double producing B cells. For example, in the $\mu$-$\gamma$ double producing B cells (mutants), it was presumed that there is no such a DNA arrangement as switch u-switch $\gamma$ ($S_\mu$-$S_\gamma$) binding. However, the present inventors could evidence the possible presence of switching by $C_x$ as well as the absence of $S_\mu$-$S_\gamma$ binding, using cells derived from a patient suffered from disgammaglobulinemia with hyper IgM.

This is also evident from the fact that the present inventors revealed that, as shown in Examples hereinafter described, $\Sigma_x$ gene in Sigma region has DNA sequences in the opposite direction to the DNA sequences contained in $\sigma_\gamma 3$ gene and $\sigma_\gamma 4$ gene, namely the DNA sequences being complimentary in mRNA level [Fig. 5(A)] as shown in Fig. 9(B).

Such complimentary sequences at mRNA level implicate the possibility of formation of secondary structures of mRNA having loop-like protrusions as shown in Fig. 5(C).

This site of mRNA with the loop-like protrusion in the secondary structure is spliced and skipped to form VDJ-$\mu$-$\gamma 3$. Furthermore, VDJ-$\mu$-$\gamma 1$, VDJ-$\mu$-$\gamma 2$ or VDJ-$\mu$-$\gamma 4$ is formed when mRNA corresponding to $C_\gamma 1$, $C_\gamma 2$ or $C_\gamma 4$ is bound in place of $C_\gamma 3$, and thus various proteins can be finally formed by translation from original mRNAs.

Furthermore, when these phenomena occur with one of the individual $C_x$ genes or with combination of more than two thereof, more variable combinations of $\mu$-$\gamma$ ($\psi e$, e, $\alpha$) are possible, which suggests the possible presence of B cells in which these splicing mechanisms work.

Furthermore, the present inventors revealed that, as shown in Fig. 6, there exists 21 DNA bp repeats in the region downstream of $\sigma_{r4}$ gene or $\sigma_{r3}$ gene and upstream of $S_\gamma 4$ or $S_\gamma 3$, respectively. More specifically, three repeats were found in the region downstream of $\sigma_\gamma 3$ gene, and 12 repeats were found in the region downstream of $\sigma_\gamma 4$ gene (further, nucleotide sequences of the repeating units are slightly different to each other). These repeating regions can also be involved in the switching described above.

The aforementioned facts will be described more in detail as follows. DNA fragments involved in the mRNA level splicing are "sigma delta core" sequence (hereinafter referred to as sdc sequence) and "sigma gamma core" sequence (hereinafter referred to as sgc sequence).

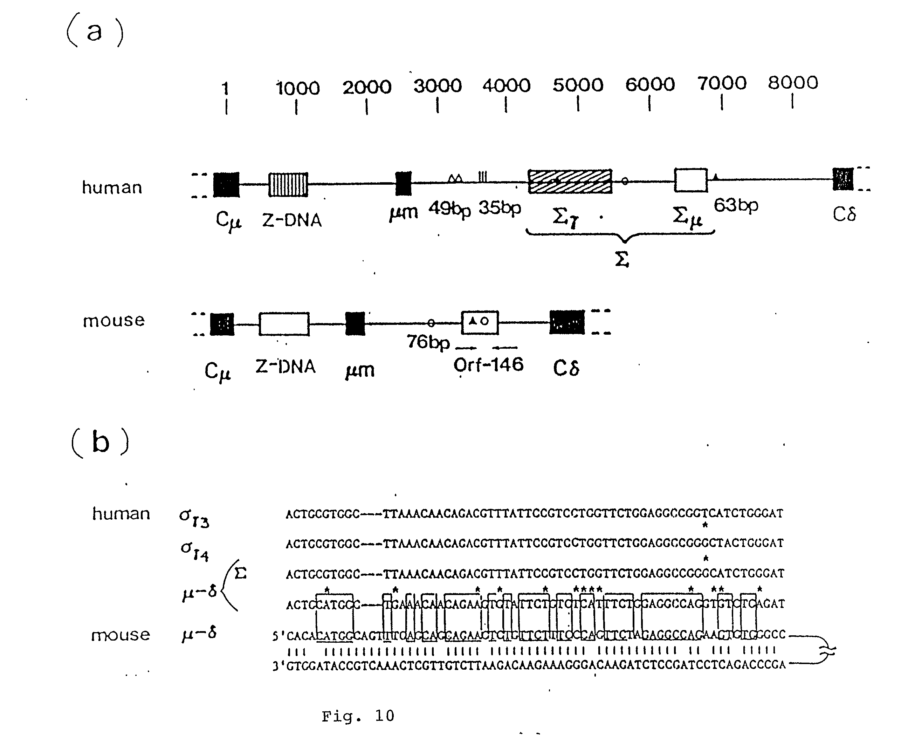

The sgc sequences are 63 bp repeating sequences at different loci in $C_\mu$-$C_\delta$ intron in human as shown in Fig. 10(a), namely in $\Sigma_x$ ($\Sigma_\gamma$) (from 4677 to 4739) and downstream of $\Sigma_\mu$ ( from 6900 to 6962), and also the sequences homologous to these 63 bp found in $\sigma_\gamma 3$, and $\sigma_\gamma 4$ [sites boxed in Fig. 9(B)].

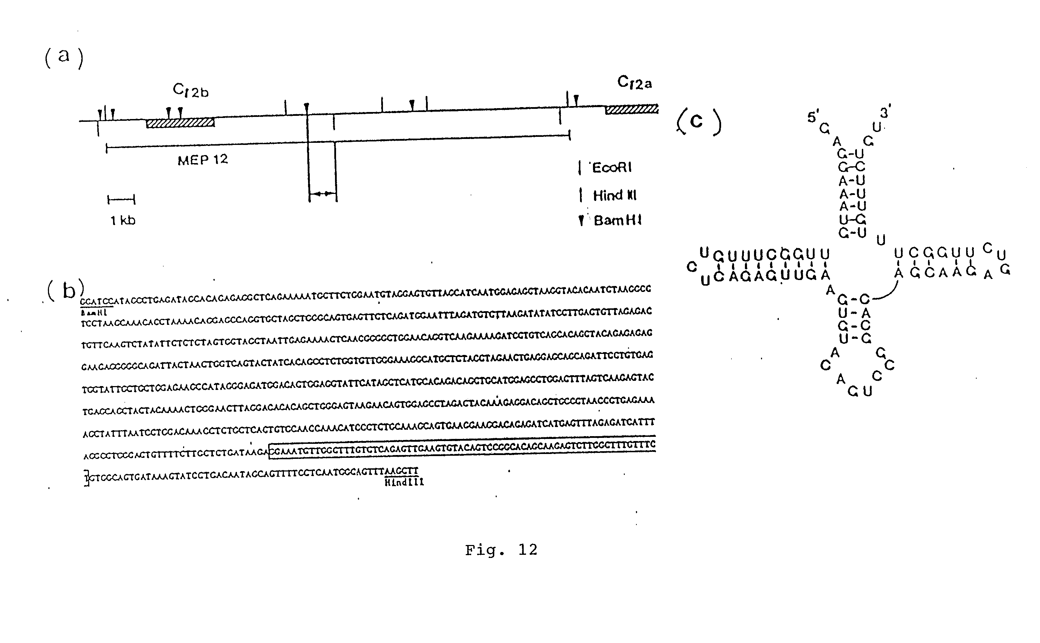

Further, a sequence having 75 % homology to the human 63 bp sequence as sgc sequence is found also in $C_\mu$-$C_\delta$ intron and in the upstream region of $C_\gamma$ gene ($C_\gamma 2b$-$C_\gamma 2a$ intron) in mouse [see Fig. 10(b) and Fig. 12 (b)]. This finding strengthens the possibility that the sgc sequence may strongly relate to expression or construction of $C_H$ gene in immunoglobulin heavy chain constant regions.

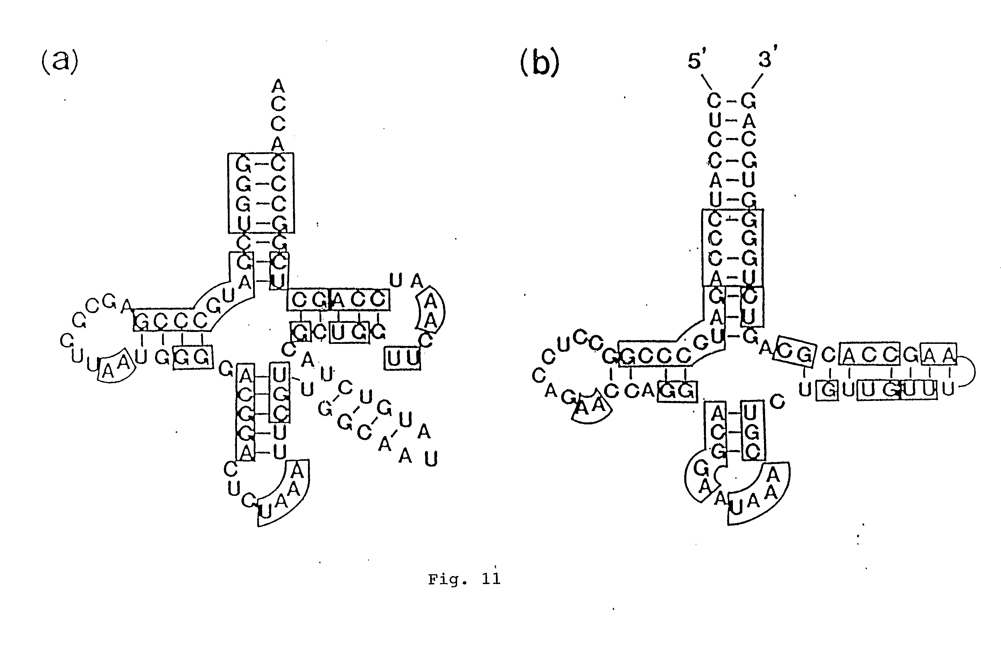

The sgc sequence is similar in size to tRNA (transfer RNA), and the RNA corresponding to this sgc sequence can form tRNA-like secondary structure, for example, as shown in Fig. 11(b). This secondary structure is very similar to the primary and secondary structures of nucleotide sequences of chloroplast tyrosyl-tRNAs shown in Fig. 11(a) (see Sprinzl, M., T. Vorderwulbecke and T. Hartmann: Compilation of sequences of tRNA genes. Nuc. Acids Res., 13, suppl. r51-r104, 1985).

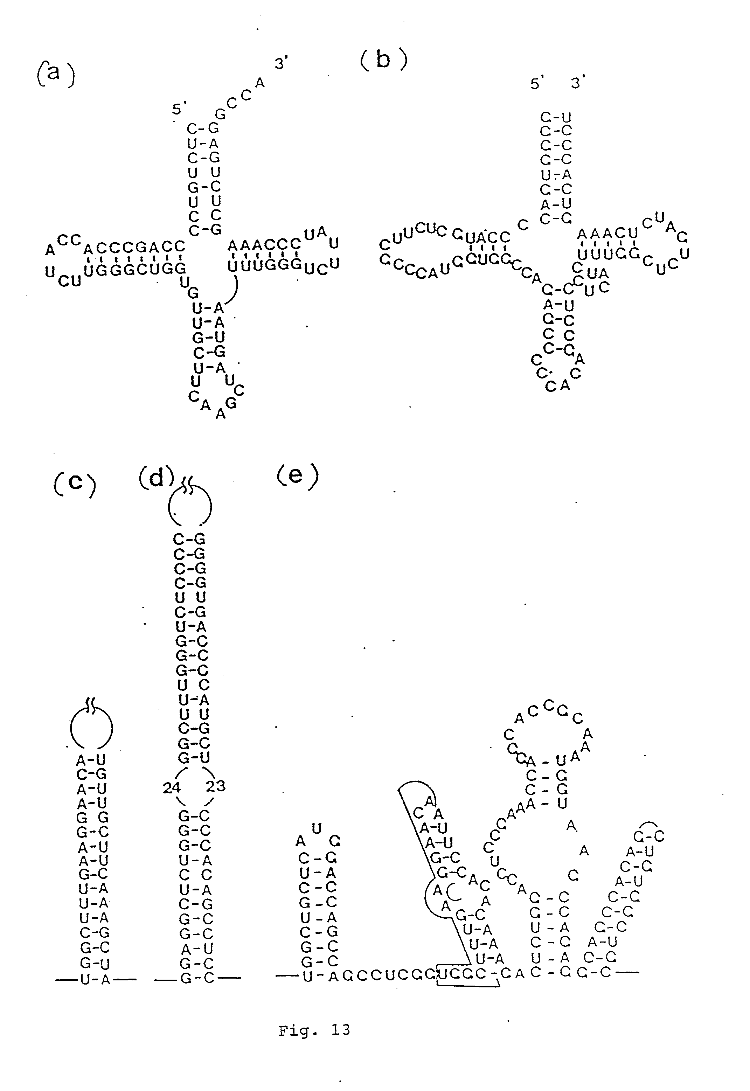

On the other hand, the sdc sequences are 76 bp repeating sequences found in mouse $\mu$-$\delta$ intron (located from 2845 to 2920 and from 3660 to 3737 having two different nucleotides) and an 81 bp sequence (from 5483 to 5563) found in human $C_\mu$-$C_\delta$ intron having analogy to said repeating sequence [see Fig. 10 (a) and Fig. 13 (b)]. The degree of similarity of these sequences (between human and mouse) is not so high, but the secondary structures of the primary transcripts from these sequences are very similar to each other. For example, both of the primary transcripts of these sequences can form tRNA-like structures (including stem-loop structures) as shown in Figs. 13(a) and (b).

The sequence complementary to this sdc sequence exists in the $\sigma_\mu$ region of $J_H$-$\mu$ intron [see Figs. 13-(c) and (d)]. This complementary sequence is located at anti-codon loop in the aforementioned tRNA-like structure, and the complementary partners in the $\sigma_\mu$ region is located in the stem-loop structure shown in Fig. 3 and Fig. 13(e).

The aforementioned sgc and sdc sequences have the following possible functions.

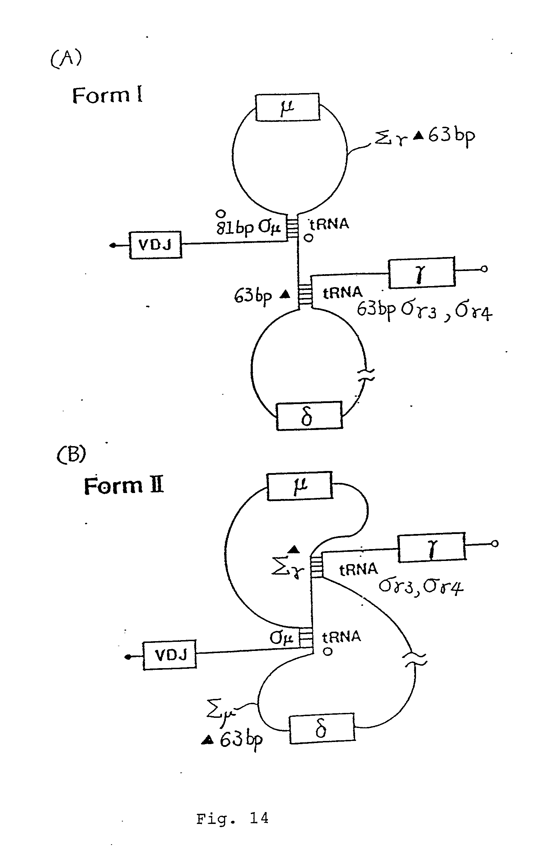

Namely, for example, as shown in Fig. 14, in the primary transcripts (RNAs) regions corresponding to sgc and sdc sequences in the long primary transcripts from $V_H$-$D_H$-$J_H$ to $\gamma$ genes, secondary hybrid structures can be formed between the tRNA-like structures derived from these sequences and the structures complementary to said tRNA-like structures.

In other words, because there exist DNA sequences complementary to the sdc sequence in $J_H$-$C_\mu$ intron, or complementary to the sgc sequence in $C_\mu$-$C_\delta$ intron, at least two kinds of secondary hybrid structures can be formed as shown in Figs. 14 (A) and (B). Furthermore, the presence of the tRNA-like structures in these secondary hybrid structures indicates that an aminoacylsynthetase-like protein may be involved in the RNA splicing. The aforementioned highly folded structure present in the $\sigma_\mu$ regions might be responsible for such RNA splicing mechanism.

In $\mu$- and $\delta$- double producing cells, $\sigma_\mu$ and its complementary sequences in $C_\mu$-$C_\delta$ intron may mediate to skip $\mu$-coding exons for direct joining of $V_H$-$D_H$-$J_H$ exon with $\delta$-coding exons. Alternative RNA splicing may be responsible for simultaneous expression of both $\mu$ and $\delta$.

In $\mu$ and $\gamma$- double producing cells, $\sigma_\mu$ and its complementary sequences in $C_\mu$-$C_\delta$ intron and, the sequence in $C_\mu$-$C_\delta$ intron and its complementary sequence upstream of $C_\gamma$ may mediate to form the aforementioned secondary hybrid structures, so that RNA splicing for $\gamma$ expression easily occurs.

By using these mechanisms, it also becomes possible to produce antibodies rich in IgG rather than in IgM.

Furthermore, it is possible to produce various proteins in addition to immunoglobulins by applying the aforementioned sdc sequence and sgc sequence which can form tRNA-like structures to the production of other proteins by genetic engineering.

## 3. Genes having promoter activity for in vitro transcription by RNA polymerase III

The present inventors found a TATA-like sequence consisting of TATAAT 300 bp upstream of the aforementioned sgc sequence and an octamer-like sequence (TTTTGCAT, which is required for expression of Ig genes) further 63 bp upstream of said TATA sequence, in the regions of $\sigma_\gamma 3$, $\sigma_\gamma 1$, $\sigma_\gamma 2$ and $\sigma_\gamma 4$, 6 kb upstream of $C_\gamma 3$, $C_\gamma 1$, $C_\gamma 2$ and $C_\gamma 4$, respectively.

The presence of these regions implicates the presence of regions having promoter activities in the regions of $\sigma_\gamma 3$, $\sigma_\gamma 1$, $\sigma_\gamma 2$ and $\sigma_\gamma 4$. In fact, as shown in Examples hereinafter when DNA fragments containing $\sigma_\gamma 3$ or $\sigma_\gamma 4$ were practically connected to pUC9 vector to synthesize transcripts by RNA polymerase (from the sensitivity to alpha-amanitin, it was shown that not RNA polymerase I and II but RNA polymerase III was involved), three major transcripts were detected, which confirmed that there existed at least two DNA regions, having promoter activity in the DNA fragments containing $\sigma_\gamma 3$ or $\sigma_\gamma 4$ since one promoter activity existed in pUC9 vector itself.

It was specified that one of the DNA regions having these promoter activities is located 230 bp upstream of sgc sequence, which is common to $\sigma_\gamma 3$ and $\sigma_\gamma 4$, the others are located 60 bp upstream of $\sigma_\gamma 3$ and 400 bp upstream of $\sigma_\gamma 4$.

The DNA regions having these promoter activities indicate possibility of discontinuous transcription, proceeding to the previously explained RNA splicing, in the simultaneous expression of two different kinds of isotypes.

Therefore, it becomes possible that the immunoglobulins of desired class or other various proteins are produced by use of gene technology method utilizing these DNA regions having promoter activity.

Construction of Sigma region which is the base of the findings of the aforementioned individual DNA fragments is considered to be strongly related to the abovementioned RNA splicing mechanisms.

Namely, Sullivan et al. (Sullivan, F. X. and T. R. Ceck: Reversibility of cyclization of the Tetrahymena rRNA intervening sequence: Implication for the mechanism of splice site choice. Cell, 42, 639-648, 1985) reported that intron removing reaction in RNA splicing is chemically reversible and that the removed introns may occasionally behave like transposons, and it has been reported that RNAs removed by RNA splicing are inserted into other RNAs by reverse reaction of RNA splicing, transformed into DNA by reverse transcriptase, and finally inserted into genome (see Sharp, P. A.: On the origin of RNA splicing and introns. Cell, 42, 397-400, 1985).

The mechanisms described in these reports may strongly relate to the formation of homologous sequences present in different sites of immunoglobulin genes, such as Sigma, $\sigma_\mu$, $\sigma_\gamma$. Namely, $\sigma_\mu$, $\sigma_\gamma$, sdc sequence, sgc sequence and the like are considered to have transposon-like characteristics.

As mentioned above, it is highly possible that Sigma region and gene groups relevant to this region which can be cloned according to the method of the present invention play important roles in mechanisms of producing class specific antibodies, especially in class switch in immunoglobulin H chains.

The following Examples demonstrate Sigma region and gene groups relevant to said region of genes derived from various haematopoietic cells or B cell lines. However, it should be understood that the use of the various haematopoietic and B cell lines themselves and the method for cloning of the individual genes are for purpose of illustration and not to limit the aspects of the present invention.

EXAMPLES

Example 1

[Identification of $\Sigma_\mu$]

DNAs are prepared according to an ordinary method from several B-cell lines (i.e., human placenta-derived germline cells, Manca, SKL7, Ly18, NALM6, Ly16, Daudi and ARA10), and then digested with HindIII to prepare HindIII-digests.

By an ordinary method, using $J_H$ gene-containing $J_H$ probe as shown in Figure 1, each of the HindIII-digests was analyzed by Southern hybridization method according to the description in "Method in gene study II, Method in biological experiments 1, a second series" (Ed. Japanese Society for Biochemistry, Tokyo Kagaku Dojin, 222-223).

Further, the DNAs were transferred onto a nitrocellulose membrane and then the membrane was exposed to X-ray film for 12 hours and 3 days for identification.

As a result, in ARA10 DNA, a relatively faint but apparently rearranged band was detected at 3.8 kb. Furthermore, in Daudi cells, a band at 3.8 kb was similarly detected.

Subsequently, the DNA fragment of the 3.8 kb band from ARA10 cells was cloned in lambda phage vector (Charon 4A vector) according to an ordinary method, and named ARAJH1.

Further, the isolation of the clone ARAJH1 was carried out by the method of Sakano et al. (Sakano, H., J. H. Rogers, K. Huppi, C. Branck. A. Traunecker, R. Maki, R. Wall and S. Tonegawa: Domains and the hinge region of an immunoglobulin heavy chain are encoded in separate DNA segments. Nature, 277, 627-633, 1979b).

Further, the 3.8 kb DNA fragment contained in the clone ARAJH1 is found also in clone 6 described below and can be obtained by digesting clone 6 with the restriction enzyme HindIII.

The DNA sequence of the 3.8 kb DNA fragment contained in ARAJH1 was identical to that located between two HindIII cleavage sites containing Sigma region in $C_\mu$-$C_\delta$ intron having a known DNA fragment (see Fig. 1).

Furthermore, using the 3.8 kb-containing ARAJH1 as a probe, Southern hybridization was carried out with HindIII-digests of DNAs of several haematopoietic cell lines (i.e., human placental germline cells, HL60, ARA10, Jurkat and Daudi) in the same manner as described above. Further, the size of bands were evaluated by lambda markers.

As a result, in addition to the 3.8 kb band found in the germline DNA, presence of at least three bands at 10, 8 and 4.5 kb in all the DNAs including the germline DNA was confirmed.

It was revealed to be quite unlikely that the 3.8 kb DNA was produced by rearrangement of $J_H$ region of ARA10 during differentiation from germline genes because it was also detected in the germline DNAs.

The reason why the 3.8 kb band was detected by $J_H$ probe in DNAs only from ARA10 and Daudi cells could have been that there were local differences in hybridization efficiencies on the nitrocellulose membrane.

However, the same membrane gave bands completely the same as those at 3.8 kb in DNAs of all the cells after the exposure to X-ray film for 3 days.

In order to elucidate the relationships between the 3.8 kb fragment originally present in the germline DNA and the other bands, the aforementioned human DNA phage library (bacteriophage lambda vector and Charon 4A were used as vectors) provided by T. Maniatis (Harvard University) was screened using this ARAJH1 as a probe, and clones hybridized with ARAJH1 were obtained by the ordinary method.

Further, the screening was carried out by the following procedure according to the method of Benton-Davis as described above.

Firstly, Escherichia coli (for example, the aforementioned E. coli DP 50, 803, ATCC 33526 and ATCC 33572 can be used) infected with the human DNA phage library of Maniatis were scattered on an L-soft agar plate in a petri dish and cultured at 37°C for at least 12 hours.

Compositions of L-agar plate and L-soft agar plate media are shown below. An L-soft agar plate was overlaid on an L-agar plate.

| Composition of L-agar plate medium: | |
|---|---|
| Bacto tryptone | 1 g |
| Bacto yeast extract | 0.5 g |
| NaCl | 0.5 g |
| Agar | 1.25 g |
| Water | 100 ml |
| (pH 7.5, adjusted with NaOH) | |

Composition of L-soft agar plate medium:

The same as L-agar plate medium except that the agar concentration is 7 %.

After completion of culture on the abovementioned plate, a dry nitrocellulose filter [88 mm, Milipore HA or BA85 (Schleicher and Schuell)] was closely placed on the L-soft agar plate so as to exclude air in the space between the plate and the filter. The filter was maintained in this state for 1 to 20 seconds and then peeled off from the plate. Thus, the plaques formed on the plate were replicated on the filter without changing their positions.

In the replication procedure, the filter and the petri dish were marked so that plaques replicated on the filter and plaques on the plate were easily collated.

Then, the filter peeled off was immersed in 0.1 N NaOH and 1.5 M NaCl solutions for 20 seconds to inactivate the phages, and furthermore immersed in a 0.2 M tris solution (pH 7.5) and 2 x SSCP [1 x SSCP (saline citrate phosphate buffer): 120 mM NaCl, 15 mM sodium citrate, 13 mM $KH_2PO_4$ and 1 mM EDTA, pH 7.2 (adjusted with NaOH)] for 20 seconds to neutralize the filter.

Furthermore, the filter was heated in vacuo at 80°C for 1.5 to 2 hours.

Then the filter thus heated was immersed in solution containing 5 x SSCP, the ARAJH1 probe labeled with $^{32}P$ by an ordinary method and 50 % formamide for 30 sec. at 42°C.

Further, the concentration of the probe was adjusted to $10^5$ to $10^6$ c.p.m. (counts per minutes) per filter.

Then the filter was immersed in 2 x SSCP at room temperature for 20 to 30 minutes, covered with a plastic rapping film, layered on a commercially available X-ray film and then allowed to stand for 24 to 48 hours. Plaques corresponding to the sensitized parts on the film, namely the plaques hybridized with the ARAJH1 probe, were identified on the plate.

Furthermore, the plaques hybridized with the ARAJH1 probe were isolated in an ordinary manner and then cloned.

Among the clones obtained by the screening, four kinds of clones inserted with fragments derived from human DNA were selected and their restriction maps shown in Fig. 1 were constructed.

The clone containing the aforementioned 3.8 kb segment itself was named clone 6, and the clone containing $J_H$ gene was named clone 3.

The other two clones, 1 and 2, are different from these clones. The regions homologous to the ARAJH1 probe in clones 1 and 2 were mapped by Southern hybridization. The results are shown in Figs. 1(b) and (c).

From these results, it was shown that clones 1 and 2 contain, respectively, 10 and 4.5 kb HindIII fragments detected by the ARAJH1 probe.

Further, the size of the HindIII fragment containing $J_H$ region is also 10 kb.

These clones were deposited in NCIMB (National Collection of Industrial and Marine Bacteria, P.O. Box 31, 135 Abbey Road, Aberdeen AB9 8DG, Scotland, UK) under the Budapest Treaty. The deposition dates and numbers are as follows:

Clone 1

Deposition date: October 6, 1988
Deposition number: 40060

Clone 2

Deposition date: October 6, 1988
Deposition number: 40061

Clone 3

Deposition date: October 13, 1988
Deposition number: 40063

Clone 6

Deposition date: October 13, 1988
Deposition number: 40064

Further, the restriction map of the upstream region of $C_\gamma 3$ in Fig. 1 is the one published by Flanagan and Rabbitts (Nature, 300, 709-713, 1982).

Furthermore, cleavage sites for HindIII, EcoRI and BamHI are indicated by marks ▼ , ▽ and ↓, respectively. Then, the obtained DNAs of clone 6 were sequenced according to two kinds of methods, i.e., the Maxam-Gilbert method (Method Enzymol., 65, 499-560, 1980) and the chain termination method (enzyme method) according to the method of Sanger et al. (Proc. Natl. Acad. Sci., USA, 74, 5463-5467, 1977) using Bluescript M13 vectors (M13mp18 and M13mp19, STRATAGENE).

The DNA nucleotide sequences obtained were compared with $J_H$-$C_\mu$ intron sequence in the downstream region of $J_H$ gene sequenced by Rabbitts et al (Rabbitts et al.: Nucleic Acids Res., 12, 6523-6534, 1984). As a result, a 442-nucleotide sequence located behind clone 6 (shown in the upper rows in Fig. 2) is 97 % homologous to the 442-nucleotide sequence(shown in the lower rows in Fig. 2) located in the upstream portion of $J_H$-$C_\mu$ intron (corresponding to the downstream region of the enhancer in H chain). Further, nucleotides that are different one another are indicated with solid circles. Furthermore, the 442-nucleotide-long sequence located behind clone 6 corresponds to the nucleotide sequence from 6387 to 6828 of the sequence reported by Rabbitts et al., but not completely identical.

The regions proved here to have homology to each other are respectively referred to as $\sigma_\mu$ - (downstream of the enhancer) and as $\Sigma_\mu$ (contained in clone 6 and located upstream of $C_\delta$ in Sigma region in the present invention).

From these results, it becomes evident that the copy sequence (analogous sequence; $\Sigma_\mu$) of the $\sigma_\mu$ (442 base pairs) in $J_H$-$C_\mu$ intron exists in other chromosome loci.

Example 2

[Identification of $\Sigma_x$ ($\Sigma_\gamma$), $\sigma_\gamma 3$ and $\sigma_\gamma 4$]

The ARAJH1 fragment obtained in Example 1 (3.8 kb) was predicted to contain a DNA fragment derived from other chromosome loci ($\sigma_x$).

Consequently, the region specified to contain $\Sigma_\mu$ in Example 1 is referred to as Sigma ($\Sigma$) region, and it was confirmed that this region contained a DNA sequence derived from $\sigma_x$ as follows.

First, to carry out chromosomal assignments of fragments detected by the ARAJH1 probe, DNAs from various kinds of mouse-human somatic cell hybrids with selectively reduced number of human chromosomes shown in Table 1 were used in Southern blots according to the method described above.

EP 0 344 319 A1

Table 1   Human chromosomes present in mouse-human somatic
cell hybrids.

Human Chromosomoe

| Hybrid Clone | 1 | 2 | 3 | 4 | 5 | 6 | 7 | 8 | 9 | 10 | 11 | 12 | 13 | 14 | 15 | 16 | 17 | 18 | 19 | 20 | 21 | 22 | X | Y |
|---|---|---|---|---|---|---|---|---|---|---|---|---|---|---|---|---|---|---|---|---|---|---|---|---|
| 1-4 |  |  | + |  |  |  | + | + |  |  |  |  | + | + | + |  | + | + | + | + | + |  | + | + |
| 1-5 | + |  |  | + |  |  | + | + |  |  | + | + |  |  |  | + | + | + |  | + | + |  |  |  |
| 1-6 |  |  | + | + | + |  | + |  |  | + | + | + |  |  | + |  | + | + | + |  |  |  |  | + |
| 2-2 |  |  |  |  |  |  | + |  |  |  |  |  | + | + |  |  | + | + | + |  | + |  | + | + |
| 3-2 |  |  |  |  |  |  | + |  |  |  |  |  | + | + |  |  | + | + |  |  |  |  |  |  |
| 3-4 |  |  |  |  | + |  |  |  |  |  |  |  | + | + |  |  |  |  |  |  | + |  | + | + |
| 7-1 |  |  | + |  |  |  |  |  |  | + |  | + | + | + |  |  | + |  |  | + | + |  |  |  |
| 1B1-24 |  |  | + |  |  |  | + |  |  |  |  |  | + | + | + |  |  |  |  |  |  |  |  |  |
| 3A6 |  |  | + | + |  | + |  | + |  |  |  |  |  |  |  | + | + | + |  | + | + |  | + |  |
| 3B5 |  |  |  | + | + |  |  |  |  |  |  |  | + | + |  |  | + | + |  | + | + |  |  |  |
| 7-1A2 | + |  | + |  |  |  | + |  |  |  |  |  | + | + | + |  | + |  | + | + |  |  |  |  |
| 7-1D2 |  |  | + |  |  |  |  |  |  |  | + |  | + | + |  |  |  |  |  | + |  |  |  |  |
| A/B7-5 | + | + |  |  | + | + |  |  |  |  | + | + |  |  |  |  | + |  |  |  |  |  |  |  |

As a result, all the bands corresponding to at least three bands which were positive to the ARAJH1 probe, and other than the 3.8 kb band in Example 1 were detected and identified on chromosome 14.

Subsequently, the restriction maps of clones 1 and 2 obtained in Example 1 were compared with the published restriction maps of constant gene loci (Nature, 300, 709-713, 1982), and it was evidenced that the maps of clones 1 and 2 are almost the same as those of the upstream regions of $C_\gamma 4$ and $C_\gamma 3$ genes, respectively.

Furthermore, clone 1 was digested with restriction enzymes EcoRI, HindIII and BamHI respectively, and the size of DNA fragments in each digest were compared.

Further, comparison of the fragments was carried out by 0.8 % agarose gel electrophoresis of the each digest mixture and staining with ethidium bromide according to an ordinary method.

Furthermore, Southern hybridization of each digest was carried out using the ARAJH1 probe according to the method described above.

The result clearly showed that clone 1 covers the region shown in Fig 1(c).

Further, the restriction map of clone 2 was the same, except for one EcoRI site, as that of the upstream region of $C_\gamma 3$ gene which had been published previously (Nature, 300, 709-713, 1982). It was therefore considered that clone 2 most likely covered the upstream region of $C_\gamma 3$ gene. Furthermore, in order to assign the loci on the chromose of the HindIII fragment (8 kb) hybridized with the ARAJH1 probe, obtained in Example 1, Southern hybridization of the EcoRI- and BamHI- digests of this 8 kb fragment with the ARAJH1 probe according to the method described above was carried out. The result suggested that the unidentified 8 kb band was located upstream of $C_\gamma 1$ gene. Furthermore, a DNA fragment corresponding to one of the 3.8 kb bands was shown to locate upstream of $C_\gamma 2$.

To determine the boundaries of Sigma ($\Sigma$) region, three probes, probes 1, 2, and 3 shown in Fig. 1(a), were prepared from the fragments of clone 6 digested with restriction enzymes indicated in Fig. 1(a).

Subsequently, Southern hybridization of clones 1 and 2 and genomic DNA with probes 1 and 3 according to the method described above detected only those fragments themselves.

Therefore, it was revealed that the mosaic structures (regions with Sigma region insertion) were restricted to the region covered by ARAJH1 and the right flanking region of 300 nucleotide HindIII-HindIII fragment.

Since it became evident from these results that the region covered by probe 2 [containing $\Sigma_\mu$ (442 base pairs) homologous to $\sigma_\mu$] was not homologous either to other regions in ARAJH1 other than probe 2 or to clones 1 and 2, it was concluded that one or more of DNA regions homologous to clones 1 and 2 must exist in ARAJH1.

Accordingly, to examine that the DNA sequences presumably located upstream of each $\gamma$ gene of the constant region genes are detected by the same DNA fragment in Sigma region, probe 4 was prepared from clone 1 fragments digested with the restriction enzymes shown in Fig. 1(c), and Southern hybridization of HindIII-digested DNA fragment of human placenta DNA was carried out using this probe 4 according to the ordinary method.

As a result, all the bands (10 kb, 8 kb, 4.5 kb and 3.8 kb) detected in Example 1 using ARAJH1 as a probe were detected by probe 4 (a part of 3.8 kb ARAJH1).

Therefore, it was proved that the ARAJH1 (3.8 kb) obtained in Example 1 consisted of two fragments (one hybridized with probe 4 and the other hybridized with probe 2).

Subsequently, Southern hybridization of clone 6 was carried out using probe 2 according to the method described above. As a result, it was found that the 5′ end region of clone 6 was also positive to the probe 2 and that clone 6 was derived from $C_\mu$-$C_\delta$ gene loci.

The restriction map of clone 6 constructed according to the ordinary method was identical, except for one HindIII site, to the published data for $C_\mu$-$C_\delta$ gene loci (Nucleic Acids Res., 12, 6523-6534, 1984).

Restriction enzyme-digested DNAs of clone 6, clone CH4-38 and clone CH4-51 were analyzed and compared in the same manner as previously used in the comparison of clones 1 and 5A using agarose gel electrophoresis, staining with ethidium bromide and Southern hybridization with the ARAJH1 probe according to the method described above.

Furthermore, DNA nucleotide sequences of fragments containing $\delta_\gamma 3$ in the upstream region of $C_\gamma 3$ and $\delta_\gamma 4$ upstream of $C_\gamma 4$ (EcoRI-HindIII, 2.5 kb DNA fragments) were respectively determined by the aforementioned Maxam-Gilbert method and the method of Sanger et al. using M13mp18 and M13mp19.

The result obtained is shown in Figs. 9(A1) to (c). The obtained result shown in Fig. 9 indicates that the Sigma region is located between $C_\mu$ and $C_\delta$ genes as shown in Fig. 1(a), which contains a complex of a DNA fragment having homology to part of $\sigma_\mu$ downstream of the enhancer and a DNA fragment ($\Sigma_x$ corresponding to the aforementioned nucleotide sequence from 4280 to 5234 in the publication by Rabbitts et al.) having homology to part of $\sigma_\gamma 3$ and $\sigma_\gamma 4$.

Furthermore, the following characteristics were found in relationships of $\sigma_\gamma 3$ gene, $\sigma_\gamma 4$ gene and $\Sigma_x$ gene having homology to part of these genes.

(1) The boundary of ending homology between $\sigma_\gamma 3$ and $\sigma_\gamma 4$ genes and the boundary of starting homology in the Sigma region are identical in $\sigma_\gamma 3$ and $\sigma_\gamma 4$.

(2) There are three 21 bp repeats between $\sigma_\gamma 3$ and $S_\gamma 3$ and twelve 21 bp repeats between $\sigma_\gamma 4$ and $S_\gamma 4$, but the sequences of these repeat units are slightly different one another.

(3) Sequences downstream of these repeat sequences have identical, each of which connects to switch sequences ($S_\gamma 3$ and $S_\gamma 4$).

(4). Polarities of 1.2 kb-long sequences are opposite between $\sigma_\gamma 3$ and $\sigma_\gamma 4$ genes, and the sites having homology to part of these genes in the Sigma regions.

The present inventors furthermore investigated $\sigma_\gamma 1$ and $\sigma_\gamma 2$ located between $\sigma_\gamma 3$ and $\sigma_\gamma 4$ of the immunoglobulin constant region genes.

Namely, the presence of $\sigma_\gamma 1$ and $\sigma_\gamma 2$ similarly having functions as $\sigma_\gamma 3$ and $\sigma_\gamma 4$ genes were identified in the regions upstream of $S_\gamma 1$ of $C_\gamma 1$ and upstream of $S_\gamma 2$ of $C_\gamma 2$, respectively, and they were cloned as in the cases of the aforementioned $\sigma_\gamma 3$ and $\sigma_\gamma 4$ genes so as to obtain clones containing $\sigma_\gamma 3$ and $\sigma_\gamma 4$, respectively.

Furthermore, nucleotide sequences (DNA sequences) of $\sigma_\gamma 1$ and $\sigma_\gamma 2$ incorporated in these clones were investigated. As a result, each of them had homology to $\sigma_\gamma 3$ and $\sigma_\gamma 4$, and had 21-bp repeats between $S_\gamma 1$ and $\sigma_\gamma 1$, and between $S_\gamma 2$ and $\sigma_\gamma 2$, respectively, as in the cases of $\sigma_\gamma 3$ and $\sigma_\gamma 4$.

From these results, the construction of the Sigma region was revealed, and it was also proved that the Sigma region had been cloned in ARAJH1.

It was shown that the Sigma region thus cloned might possibly play an important role in class switch of immunoglobulins, and cloning of the Sigma region or the gene group relevant to this region was shown to be extremely useful to provide technology or agents necessary to elucidate class switch of immunoglobulins. As mentioned above, it is also useful for establishing technology necessary to control kinds of antibody subclasses as desired.

Example 3

[Rearrangement at DNA level]

Investigation of functions of the Sigma region or the gene group relevant to this region revealed in Examples 1 and 2 suggested possible presence of DNA rearrangement, for example, as shown in Fig. 7.

Accordingly, DNAs were prepared by the ordinary method from human placenta-derived germline cells, HL60, ARA10, Jurkat and Daudi, and then EcoRI-digests were obtained. The digests obtained were fractionated by agarose gel electrophoresis and further subjected to Southern hybridization with the ARAJH1 probe.

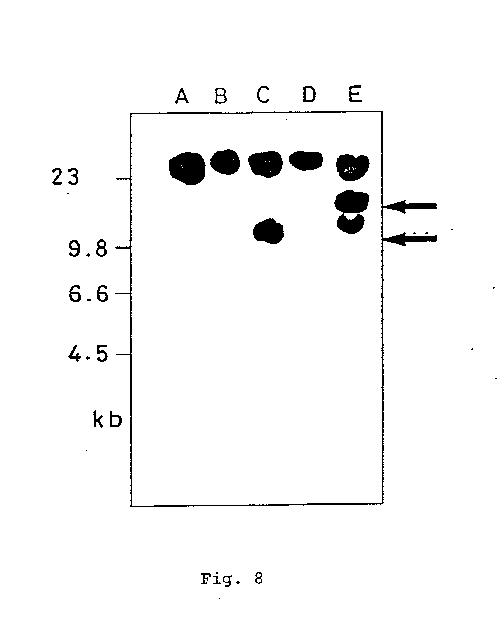

As a result, as shown in Fig. 8, a band hybridized with ARAJH1 was detected at 28kb in all the cells, and in addition, one band was detected at 9.8 kb in ARA10 cells, and two bands were detected in Daudi cells, one at 9.8 kb and the other between 9.8 kb and 23 kb.

This result suggested the presence of rearrangement of H chain genes due to deletion in $C_\delta$ region.

Consequently, it becomes possible to clone these cells assumingly rearranged and to construct hybridomas by fusion with myeloma cells so as to obtain desired antibodies having H chain structures corresponding to u or specific $\gamma$ subclass.

Example 4

[Confirmation of the presence of sgc sequence in mouse immunoglobulin genes]

Firstly, using a DNA synthesizer (Applied Biosystem Inc.), a DNA fragment consisting of 66 mer [Fig. 10(b)] having a sequence corresponding to a 63 bp-DNA sequence (sequence having homology to part of human sgc) found in human $C_u$-$C_\delta$ intron was chemically synthesized.

Then, using the resultant 66 mer as a probe, Southern hybridization of clone MEP12 (Roeder, W., R. Maki, A. Traunecker and S. Tonegawa: Linkage of the four r subclass heavy chain genes. Proc. Natl. Acad. Sci. 78, 474-478, 1981)) was carried out.

Further, the restriction map of clone MEP12 is shown in Fig. 12(a).

As a result of the aforementioned hybridization, the presence of several different regions giving positive signals to the 66 mer probe was proved in $\gamma_{2b}$-$\gamma_{2a}$ intron. Among the regions giving positive signals, the 860 bp BamHI-HindIII fragment (the site indicated with ↔ in Fig. 12(a)) located 10 kb upstream of $C_{\gamma}2a$ gene gave the strongest signal. The analysis of the nucleotide sequence of this BamHI-HindIII fragment (860 bp) gave the result shown in Fig. 12(b). By determination of the sequences of this 860 bp fragment, the sequence (the boxed 69 bp sequence) having homology to part the aforementioned 66 mer probe was found. A primary transcript from this 69 bp sequence could form the aforementioned tRNA-like structures.

These results indicated that, in upstream regions of $C_{\gamma}$ genes in both mouse and human, there are DNA sequences (sgc sequence) whose primary transcripts can form tRNA like structures.

Example 5

[Detection of promoter activity in $\sigma_{\gamma}$ region]

Transcription mixtures were prepared from Hela cell extracts according to the procedure described by Dignam et al. (Dignam, J. D., R. M. Lebovitz and R. G. Roeder: Accurate transcription initiation by RNA polymerase II in a soluble extract or isolated mammalian nuclei. Nucl. Acids Res., 11, 1475-1489, 1983) and used in vitro synthesis of transcripts (RNAs) independently using the template DNAs shown below. Further, a template concentration of 50 micrograms/ml was used.

Template DNA

(a) BamHI-digested pSG4
(b) BamHI-digested pSG3
(c) DNA containing RNA polymerase I promoter [provided by Dr. M. Muramatsu (Tokyo University)]
(d) Adeno VA DNA containing RNA polymerase III promoter
(e) HindIII-digested pSG3
(f) HindIII-digested pSG4

Further, the abovementioned recombinant plasmids pSG3 and pSG4 were prepared by recloning the EcoRI-HindIII fragments containing $\sigma_{\gamma}3$ and $\sigma_{\gamma}4$ obtained in Example 2 in pUC9 according to the ordinary procedure including steps such as fractionation by electrophoresis and reaction using ligase.

Further, when the abovementioned template DNAs (a) to (d) were used, alpha-amanitin concentrations used were 0, 1, 50 and 250 micrograms/ml.

After completion of incubation for RNA synthesis reaction, the synthesized RNAs were extracted with a phenol-chloroform mixture, and after treatment with glyoxal, subjected to 1.8 % agarose gel electrophoresis. RNAs fractionated were stained for analysis. Further, DNAs used as size markers were also treated with glyoxal.

The following results were obtained by this electrophoresis.

In the case where the BamHI-digested pSG4 (a) was used, bands were detected at least at 600 bp, 970 bp, 1150 bp, 1400 bp, 2000bp and 2200 bp at alpha-amanitin concentrations of 0 and 1 microgram/ml; at an alpha-amanitin concentration of 50 micrograms/ml; a band at 600 bp only was detected, and further with an increased concentration of alpha-amanitin, i.e., at 250 micrograms/ml, no band was detected.

In the case where the BamHI-digested pSG3 (b) was used, the result was similar to those of the above-described case where BamHI-digested pSG4 (a) was used, except that bands at 600 bp and 1400 bp were detected with alpha-amanitin at 50 micrograms/ml.

Furthermore, in the case where the DNA containing RNA polymerase I promoter (c) was used, bands were consistently detected at least at 800 bp independently to alpha-amanitin concentrations.

On the other hand, in the case where Adeno VA DNA containing RNA polymerase III promoter (d) was used, a band was detected at 150 bp at alpha-amanitin concentrations of 0, 1 and 5 micrograms/ml, but this band at 150 bp was not found at an alpha-amanitin concentration of 250 micrograms/ml

Furthermore, in the case where HindIII-digested pSG3 (e) and HindIII-digested pSG4 (f) were independently used, bands were detected at least at 1000 bp, 1600 bp, 1800 bp, 2150 bp and 2500 bp.

From those results obtained by       , it was shown that there were regions having promoter activity in pSG3 and pSG4, and that regions downstream of the regions having promoter activities in pSG 3 and pSG 4 were transcribed by these promoter actitivies to synthesize RNAs.

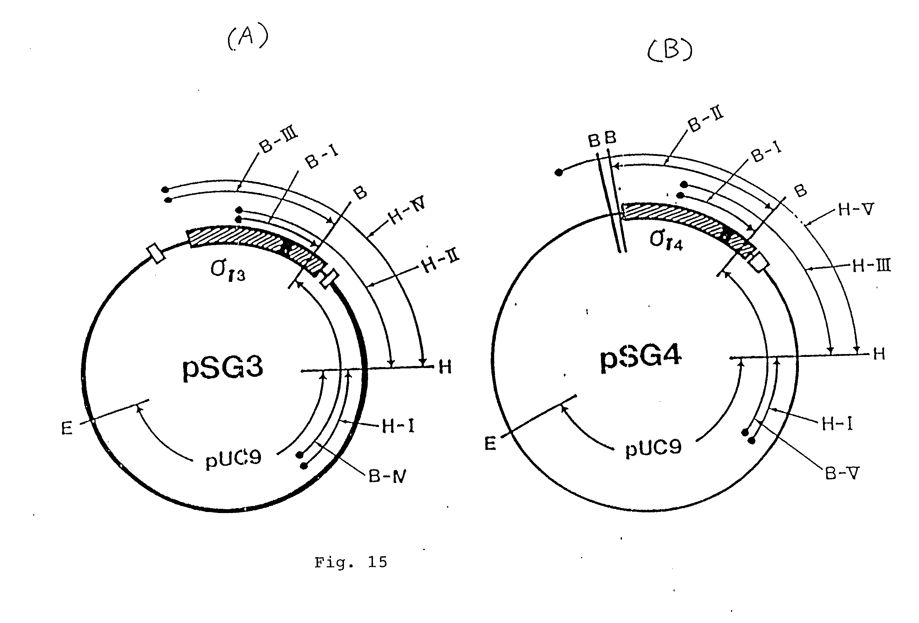

Numbers of nucleotides in the individual RNAs obtained were compared with those in structures of pSG 3 and pSG 4 indicated in Fig. 15. As a result, the RNAs synthesized were revealed to correspond to the sequences, B-I to B-V and H-I to H-V, having the following numbers of nucleotides.

| B-I: | 600 bp | B-II: | 970 bp |
|------|--------|-------|--------|
| B-III: | 1150 bp | B-IV: | 2000 bp |
| B-V: | 2200 bp | H-I: | 1000 bp |
| H-II: | 1600 bp | H-III: | 1800 bp |
| H-IV: | 2150 bp | H-V: | 2500 bp |

Furthermore, three major promoter active sites as indicated by solid circles in Fig. 15 were identified by analyzing these results.

One of the sites were located in pUC9 vector per se.

In fact, in order to confirm the presence of the promoter region, the RNA synthesis was carried out in the same manner as described above using pUC9 vector only. As a result, it was proved that one promoter region existed in the pUC9 vector, located in the site shown in Fig. 15.

On the other hand, one of the other promoters was located 230 bp upstream of sgc sequence (the sites indicated by ■ marks in $\sigma_\gamma 3$ and $\sigma_\gamma 4$ in Fig. 15), which is common to $\sigma_\gamma 3$ and $\sigma_\gamma 4$.

Furthermore, the other promoters were located upstream of $\sigma_\gamma 3$ and $\sigma_\gamma 4$, respectively.

Besides, the sensitivity to alpha-amanitin in RNA synthesis was the same as expected for RNA polymerase III when pSG 3 and pSG 4 were used as samples, which suggested that the promoter activity found in pSG 3 and pSG 4 was mediated by RNA polymerase III.

Furthermore, the clone containing $\sigma_\gamma 1$ or $\sigma_\gamma 2$ which had been previously cloned was recloned in pUC9 as described above, and the presence of promoter activity was examined in the same manner as described above. As a result, the same kind of promoter activity as found in $\sigma_\gamma 3$ or $\sigma_\gamma 4$ was also observed in $\sigma_\gamma 1$ and $\sigma_\gamma 2$.

Example 6

Examination for serum immunoglobulins was carried out with 300 and more of myeloma patients, and four patients who produced relatively large numbers of IgD were selected.

Then, the bornmarrow or peripheral blood was collected from each patient and the lymphocyte fraction was separated and DNAs was prepared according to an ordinary emthod.

The DNAs were then treated with HindIII to prepare HindIII digests.

The HindIII digests thus obtained were subjected to Southern hybridization, which revealed that DNA rearrangements had occurred between $\sigma_\mu$ and $\Sigma_\mu$ in the lymphocyte fractions described above.

Namely, antibody producing cells or B cells producing IgD-rich antibodies which virtually experienced the mechanism of DNA rearrangement described above in detail were isolated.

Further, otherwise specified, hybridization procedures in the Examples above were carried out by the method of Wahl et al. (Wahl, G. M., M. Stern and G. R. Stark: Efficient transfer of large DNA fragments from agarose gels to diazobenzyloxymethyl paper and rapid hybridization using dextran sulfate. Proc. Natl. Acad. Sci., 76, 3683-3687, 1979).

REFERENCE TO THE DEPOSITION OF MICROORGANISMS

The clones described below were deposited in NCIMB (National Collection of Industrial and Marine Bacteria, P.O. Box 31, 135 Abbey Road, Aberdeen AB9 8DG, Scotland, UK) under the Budapest Treaty, and the deposition dates and numbers are as follows:

Clone1

Deposition date: October 6, 1988
Deposition number: 40060

Clone 2

Deposition date: October 6, 1988
Deposition number: 40061

Clone 3

Deposition date: October 13, 1988
Deposition number: 40063

Clone 6

Deposition date: October 13, 1988
Deposition number: 40064

**Claims**

1. A recombinant DNA of human immunoglobulin heavy chain genes, comprising $\sigma_\mu$ gene which is an intron DNA sequence located between an enhancer sequence downstream of J region and a switch sequence upstream of $C_\mu$ gene ($S_\mu$).

2. A recombinant DNA as set forth in claim 1, wherein said $\sigma_\mu$ gene is cloned in lambda phage.

3. A recombinant DNA of human immunoglobulin heavy chain genes containing $\Sigma_\mu$ gene, which has homology to part of $\sigma_\mu$ gene that is an intron DNA sequence located between an enhancer sequence downstream of J region and a switch sequence upstream of $C_\mu$ gene ($S_\mu$) and is located between said $C_\mu$ gene and $C_\delta$ gene.

4. A recombinant DNA as set forth in claim 3, wherein said $\Sigma_\mu$ gene is cloned in lambda phage.

5. A recombinant DNA containing $\sigma_x$ gene present further upstream of a switch sequence located upstream of constant region genes $C_x$ wherein x is $\gamma_3$, $\gamma_1$, $\psi_\epsilon$, $\alpha_1$, $\gamma_2$, $\gamma_4$, $\epsilon$ or $\alpha_2$ coding for a heavy chain which is a component of human immunoglobulin IgX (wherein X corresponds to $G_3$, $G_1$, $\psi E$, $A_1$, $G_2$, $G_4$, E and $A_2$, respectively).

6. A recombinant DNA as set forth in Claim 5, wherein said $\sigma_x$ gene is cloned in lambda phage.

7. A recombinant DNA as set forth in claim 5, wherein said $\sigma_x$ gene is $\sigma_\gamma 3$ gene cloned in a plasmid.

8. A recombinant DNA as set forth in claim 5 which contains an EcoRI-HindIII DNA fragment containing $\sigma_\gamma 3$ gene obtained by treating human immunoglobulin heavy chain genes with restriction enzymes EcoRI and HindIII.

9. A recombinant DNA as set forth in claim 5, wherein said $\sigma_x$ gene is $\sigma_\gamma 4$ gene cloned in a plasmid.

10. A recombinant DNA as set forth in claim 5 which contains an EcoRI-HindIII DNA fragment containing $\sigma_\gamma 4$ gene obtained by treating human immunoglobulin heavy chain genes with restriction enzymes EcoRI and HindIII.

11. A recombinant DNA as set forth in claim 5, wherein said $\sigma_x$ gene is $\sigma_\gamma 1$ gene cloned in a plasmid.

12. A recombinant DNA as set forth in claim 5 which contains an EcoRI-HindIII DNA fragment containing $\sigma_\gamma 1$ gene obtained by treating human immunoglobulin heavy chain genes with restriction enzymes EcoRI and HindIII.

13. A recombinant DNA as set forth in claim 5, wherein said $\sigma_x$ gene is $\sigma_\gamma 2$ gene cloned in a plasmid.

14. A recombinant DNA as set forth in claim 5 which contains an EcoRI-HindIII DNA fragment containing $\sigma_\gamma 2$ gene obtained by treating human immunoglobulin heavy chain genes with restriction enzymes EcoRI and HindIII.

15. A recombinant DNA containing $\Sigma_x$ gene, which has homology to part of one or more of $\sigma_x$ genes present further upstream of a switch sequence located upstream of a constant region gene $C_x$ (wherein x is $\gamma_3$, $\gamma_1$, $\psi_\epsilon$, $\alpha_1$, $\gamma_2$, $\gamma_4$, $\epsilon$ or $\alpha_2$) coding for a heavy chain which is a component of human immunoglobulin IgX (wherein X corresponds to $G_3$, $G_1$, $\psi E$, $A_1$, $G_2$, $G_4$, E and $A_2$, respectively), and is located between $C_\mu$ gene and $C_\delta$ gene.

16. A recombinant DNA as set forth in claim 15, wherein said $\Sigma_x$ gene is cloned in lambda phage.

17. A recombinant DNA as set forth in claim 15, wherein said $\Sigma_x$ gene has homology to part of $\sigma_\gamma 3$ gene, $\sigma_\gamma 1$ gene, $\sigma_\gamma 2$ gene and $\sigma_\gamma 4$ gene as said $\sigma_x$.

18. A recombinant DNA, comprising sigma gamma core sequence consisting of a 63 bp repeating sequence, located in $\Sigma_x$ gene as set forth in claim 15 and downstream of $\Sigma_\mu$ gene as set forth in claim 3, of human immunoglobulin heavy chain genes; a 63 bp sequence which has homology to part of said 63 bp sequence and is contained in $\sigma_\gamma 3$ gene, $\sigma_\gamma 1$ gene, $\sigma_\gamma 2$ gene and $\sigma_\gamma 4$ gene, respectively; or a 66 bp sequence, which has homology to part of said 63 bp sequence and is located between $C_\mu$ gene and $C_\delta$ gene of mouse immunoglobulin heavy chain genes.

19. A recombinant DNA containing sigma delta core sequence consisting of a 76 bp repeating sequence existing in the intron between $C_\mu$ gene and $C_\delta$ gene, which has a complementary sequence in $\sigma_\mu$ gene which is a DNA sequence of intron located between an enhancer sequence downstream of J region of mouse immunoglobulin heavy chain genes and a switch sequence upstream of $C_\mu$ gene, or an 81 bp sequence having analogy to part of said 76 bp sequence, which has a complementary sequence $\sigma_\mu$ gene and is located in the intron between $C_\mu$ and $C_\delta$ gene, of human immunoglobulin heavy chain genes.

20. A method for producing proteins using RNA trans-splicing with the use of the sigma gamma core sequence as set forth in claim 18 or the sigma delta core sequence as set forth in claim 19.

21. A method for producing proteins using a promoter activity in an EcoRI-HindIII DNA fragment containing $\sigma_\gamma 3$ gene as set forth in claim 5, wherin human immunoglobulin heavy chain genes are treated with restriction enzymes EcoRI and HindIII.

22. A method for producing proteins using a promoter activity in an EcoRI-HindIII DNA fragment containing $\sigma_\gamma 4$ gene as set forth in claim 5, wherein human immunoglobulin heavy chain genes are treated with restriction enzymes EcoRI and HindIII.

23. A method for producing proteins using a promoter activity in an EcoRI-HindIII DNA fragment containing $\sigma_\gamma 1$ gene as set forth in claim 5, wherein human immunoglobulin heavy chain genes are treated with restriction enzymes EcoRI and HindIII.

24. A method for producing proteins using a promoter activity in an EcoRI-HindIII DNA fragment containing $\sigma_\gamma 2$ gene as set forth in claim 5, wherein human immunoglobulin heavy chain gene is treated with restriction enzymes EcoRI and HindIII.

25. A method for producing proteins using RNA trans-splicing with the use in combination of sigma gamma core sequence as set forth in claim 18 and/or sigma delta core sequence as set forth in claim 19 and one or more of the promoter activities in the EcoRI-HindIII DNA fragment as set forth in claims 21, 22, 23 or 24.

26. A method for obtaining a recombinant DNA as set forth in claims 2, 4, 6 or 16, comprising the steps of preparing a HindIII fragment from haematopoietic cell DNA and selecting from the resultant HindIII fragment a 3.8 kb DNA fragment which can be detected by a probe containing human immunoglobulin heavy chain $J_H$ gene for cloning, and then screening a clone which hybridizes with said 3.8 kb DNA fragment from a human DNA phage library using said DNA fragment as a probe.

27. A method for producing antibodies, comprising a step in which antibodies are produced in cells of a cell line capable of expressing human immunoglobulin heavy chain genes lacking $C_\delta$ gene.

28. A method for producing antibodies as set forth in claim 27, wherein said cell line is ARA10 or Daudi cell.

29. A method for producing antibodies, comprising a step in which antibodies are produced in cells of a cell line capable of expressing human immunoglobulin heavy chain genes lacking $C_\mu$ gene and $C_\delta$ gene.

30. A method for producing antibodies as set forth in claim 29, wherein said cell line is ARA10 or Daudi cell.

EP 0 344 319 A1

Fig. 1

ATCAATTAAATCAGATAAATAAATCATTACCCTGAATTATTCCAGTTAAGCATGTTAGTTGGTGGCACAAGAGAAAACCCAGTCAGACAGTGCTGAAGAC
ATAAGTTTTAGGTAAAATGTGCATCATTATCCTGAATTATTTCAGTTAAGCATGTTAGTTGGTGGCATAAGAGAAAACTCAATCAGATAGTGCTGAAGAC

AGGACTGCGGAGACACCCCAGAAGGACAGATTCTGTTCCGAATCACTGATGCGGCGTCAGCAGGACTGGCCTAGTGGAGGCTCTGGGAGGGTGGCTGCCA
AGGACTGTGGAGACACCTTAGAAGGACAGATTCTGTTCCGAATCACCGATGCGGCGTCAGCAGGACTGGCCTAGCGGAGGCTCTGGGAGGGTGGCTGCCA

GGCCCGGCCTGGGCTTTGGGTCTCCCCGGACTACCCGGAGCTGGGATGCGTGGCTTCTGCCGCCGGACCGACTGGCTGCTCAGGCCCCAGCCCTTGTTAA
AGCCCGGCCTAGGCTTTGGGTCTCCCCGGACTACCCAGAGCTGGGATGCGTGGCTTCTGCTGCCGGGCCGACTGGCTGCTCAGGCCCCAGCCCTTGTTAA

TGGACTTGGAGGAATGATTCCATGCCAAAGCTTTGCAAGGTTCGCAGTGACCAGGCACCCGACATGGTAAGAGACAGGCAGCCGCCGCTTGC-GCATTCA
TGGACTTGGAGGAATGATTCCATGCCAAAGCTTTGCAAGGCTCGCAGTGACCAGGCGCCCGACATGGTAAGAGACAGGCAGCCGCCGC-TGCTGCATTTG

HindIII

CTTCTCTTAAAACTTTGTATTTGATGTCTTATTTCCACTAGAAGGCGAATTGGTCTTAATTGCTTCTCTTAAGCCACCACTTCTAAAGCCAAAATCTGTT
CTTCTCTTAAAACTTTGTATTTGACGTCTTATTTCCACTAGAAGGGGAACTGGTCTTAATTGCTTGATGAAGAGCAGGAGACTCATTTATGTGAGTCTTT

Fig. 2

EP 0 344 319 A1

Fig. 3

EP 0 344 319 A1

Fig. 4

Fig. 5

Fig. 6

Non-homologous     σ     Switch    Constant region

$\gamma_3$

$\gamma_1$

$\gamma_2$

$\gamma_4$

21bp – repeat

Fig. 7

$\sigma\mu$    $\Sigma$    $\sigma_{\gamma3}$   $R_{\gamma3}$

VDJ | $C\mu$ | $C\delta$ | $C_{\gamma3}$

$S\mu$    $\Sigma_x$   $\Sigma\mu$    $S_{\delta3}$

VDJ | $C\mu$ | $C_{\gamma3}$

$S\mu$    $S_{\gamma3}$

EP 0 344 319 A1

Fig. 8

# (A1)

EP 0 344 319 A1

5'-flanking sequence of $\sigma_{\gamma3}$

GAATTCTATTCTGCCATGAAAAAGAATGAAATCTTGTCATCTGAGGTAACATGGATGAGCTTGGAGGACATGTTAAGTGAAATAAAGCTAGACACAGACAGCCAAATATCACATGTTCTC
EcoRI

ACTCATATATCGAAGCCAAATAAGTTGATCTGATAGAAATAGGGAGTAGAATAGTGGTAACCAGAGGCTGGGAATGGGAGGGGATAGGATAGCTAGAAGTCGATTAATAAATAAAAAATT

ACAGTCACGTACGACGAATAGGCCCCGGTGTTCTCTAACACCATAGGGTGACTATAACTAACACCAATTTATTGTATATTTTCATACGGCTAGAAGAGCAAATTTTGATTGTTCCCAGCA

CAAAGAAGTGATAAATGTTTGAGGTGATGGATATGCTAATTGTTGCAGGAAGTCAGGGACCCCGAATGGAGGGACCAGCTGGAGCCATGGCAGAGGAACATAAATTGTGAAGATTTCATG

GACATTTATCAGTTCCCAAATAATACTTTTATAATTTCTTATGCCTGTCTTTACTTTAATCTCATAATCCTGTTATCTTCATAAGCTGAGGATGTACGTCACCTCAGGACCACTGGGATA

ATTGTGTTAACTGTACAAATTGATTGTAAAACATATGTGTTTGAACAATATGAAATCAGTGCACCTTGAAAAAGACAGAATAACAGCGATTTTTAGGGAATAAGGGAAGACAACCATAAG

GTCTGACTGCCTGAGGGGTTGGGCAAAAAGAGCCATATTTTTCTTCTTGCAGAAAGCCTATAAATGAACGTGCAAGTAGGGAAGATATCGCTAAATTATTTTCCTAGCAAGGAATATTAA

TACTAATACCCTGGGAAAGGAATGCATCCCTGGGGGAGGTCTATAAACGGCCGCTCTGGGAATGTCTGTCTTATGTGGTTGAGATAAGGACTGAGATAAGGACTGAGATACGCCCTCGTC

TCCTGCAGTACCCTCAGCTTATTAGGGGGGTGAAAAACTCCACCCTGGTAAATTTGTGGTCACACTGGTTCTCTGCTCTCAACTCTGTTTTGTGTTGTTTAAGATGTTTATCAAGATAAT

ATATGCACTGCTGAACA   TAGACCCTTATCAGTAGTTCTGTTTTTGCCCTTTG-CCT----------------TGTGATCTTTGT

                TGGACCCTTATCAGTGGTTCTGCTTTTGCCCTTTGTCCTGTTCCCTCAGAAGCATGTGATCTTTGT

          TAGACCCTTATTAGTAGTTCTGCTTTTTGCCTTTG--------------AAGCATGTGATCTTTGT   ACCCACTCCCTGTGCTTACACCCCCTCCCTTT

TTGAAACCCTTAACAAAAAACCTGCTGGTTTGAGGCTCAGGCGGTCATCACAGTCCTACTGATATGTGATGTCACCCCCGGCTGCCCAGCTCTAAAATCCTCTCTTTATACTGTCTCTCT

TTATTTCTCAGCTCGGCCAACAATTATGGAAAACAGAAAGAACCTACATTGAAATATTGGGGGCAGGTTCCCCCAATACTAATTATCCTGATTTGATCATCACCCATTGTATATATGTATC

CAAATATCACAATGTACCCCAAAATATATACAATTATTATGTGTCAATTAAAAACAATCATAAAACTTTTAAACAGCTAAAATAAAGTATATTGTTTTCTTCAAAAAAAATCTAATGCCA
$\sigma_{\gamma3}$

Fig. 9

(A2)

5'-flanking sequence of σγ4

GAATTCTAAGTACTACACTTACATAATTGATTCAGGAATGCTAAAAGGAGTTCATAGATGCAAAACTGGCCTTTTCCCTGGAAGATGAGGAGCAATTCATTGTCCTTCCAAAGATGACAA
<u>EcoRI</u>

CTTGAATTTCTACCAACTCAAAAGAGCTTTTGCATTGCTATCAATTATGTACAACTTAGAGCAGTGGTCCCCAACATTTTTGGCACCAGGAACCAGTTCCATGGAAGACAATTTTTCCAC

AGACCACGATCGGGGGATGGCTTGGGGACAAGCTGTTCCACCTAGATCATTAGGCATTAGGGTGTCATAAGGAGGTGTGCACTTAGATCCCCGGGAATGTGCGGCTCGCAATAGGGTTCGC

TCCTGTGAGAATCTAATGCTGCCACTGATCTGACAGGAGGTGGAGCTCGGGCAGGAATGCTCACACACCCCTCACCTCCTGCTCTGTGGCCCAGTTCCTAACAGGCCATGAACCGGTTCC

AGTGCATGACCCACGGATTGGGGACCCCTGGCTTATAGAGGTGTAAAATAGTTCAAAGGAAATAAAAGATGCAGAGCTCCACAGAATGAAATAACCTGGAAGAGTGTACAAGACGATCCC

TTGCTTTCCATGGAAGGCACCTACTAATTTTTCTCAATGTTTCCTATAAACATATATAACTGACTGACAGAAACAGATCCATAATATAAAGAAGACCCCTGTAAACCAATGAGAAAAAA

ATCAAATAATCCAACGACGGAATACGCAAGAGAATTGAACAGATGTTTTACAGAAGATATCCAAATAGCCACTAAACATATGAAAGGTGTTGAACCACACTAGTCAACAGGGAAATGAAA

ATGAAAAACCACATGAGAGAAAGTAGTTCTGATTCCAGTAATGCTGGAGCAGCTAATATCAGACTAGCCCTTTGGCAGATGGCAATTATAAACACTGGAAACACTGTAAGCACACACAAC

ACCCACACACACCAATTGCCAGGCACTGGAACATGACCAGAAGTAGGCAAACACTAGTAAGGATTATTCCGTGAAATATTCGTCTGAAGTCACACCCCAGTGCATGTAATGGGTGCAGCTA

CAGTTTAAGCAGGAAACTGCAGCCCTCCTGGTGAGGAGTGGGATGCAGGGCTGCATTTTCAGAGCAGCTGGAAATGAAGAGAAGATGTCCATAAAGGAGAAGCTCACCGAAGGAAACCAC

ACAATCTGCAAGTAAACTCCACTGAAACCTCTGGCCGATCCCTTAGGTGTGCATGGGTAGGGAAAACTCCAAAGGGCCCAGCAGAAAGCAACACCTGTAAGTCGACAGAACTGAGATTCC

AGCTACTGCCAACTGCCAGGCAGACAGACAGACTTGGGAGTTTGAGTCAACTCAAGCTAACTGCTAACATTGAAAAACAATTAATGCTCTGCTAAGAAAGAATGCAAACCCCATAGCCTG

TACGCATGTTATCAACATCAGGTGCACATCCAAAATTACCATGGATGCAAAGAAACATGAAAATGTGATCCATAGTCAGAAGAAAAAGGGATCAATGGAGATCAACTCCAAGATGACCTA

GATGCAGATACAGTTATCAGACAAGGACTTTAAAGAAGTTATGTTAAACATGTTCAAAGACTTAAAGGAAAATACTATTATAATGAGTGACTAGATGGGGAATCTCTATAAGAGACGGAA

AATATTTACAAGAACCAAATGAAAATTCTAGAACTGAAAGTATGATTCTGAAATGAAAAACATCATTTTTCAGAGCAGGGTGAGAATGGACATGGGACTCAGAGCTGAGCAGGCCTCGTG

CGCCCCACGAGGGAGACACAGACGACTGGGGGATTTCAAGGCTGGCAGAGGCCAGAGATGGATCCCCAGCTGGGACTGGACCTGGGCTTATGGGAGCAACAGGTGACCCATCCTCCTTCC
<u>BamHI</u>

TCGGCGCCCACCCTGCCCGGCCCCTCCAGCCCAGCACAGGCATTGGATAGAACCGGGAGAGAGCAGGCCAGGCACTGAGGCCTCTGCCCCAAATGCCCACAGCCTGGGGAAAATGAGCAG

ATAGATGGGCGGGGCAGTGGATCCCCCAGGCACACCCACACAGTGCCACACAGCCCCACTTGGGCCAGAGGGGGCAGGAGGCTCGCCACCCCTCCTGTGGTTTCTCCCACACTTGATGCAGC
<u>BamHI</u>                                                                                                                                    σγ4

EP 0 344 319 A1

Fig. 9

Fig. 9

(B)

EP 0 344 319 A1

```
                                  *        *   *        *            *              * *        *
μ-δ  GAGGATGAGCCTTGAGCCTTAAATCTAATGCAGCTTTCCCTGCTGGGTTT-GGGCTTGCTTGGGACCCATGGCTCCTCTCTCCCTTTCTATGTATCCCTTTTAGAATACGAATGTCCATC
γ3   TATATTGTTTTCTTCAAAAAAAATCTAATGCAGTTTTCCCTACTAGGTTTTGGGCTTGCTTAGGACCCATGGCTCCTCTCTCCCTTCCAATGTATCCCTTTTGGAATACGAATGTCCATC
                     └────────→ σγ3                                                      *           * *        *

μ-δ  CTATGCCTGCCCCATCATTGTACTTTGGAAGCAGATAACTTCTTGTCAAGTTACAAAGGTCCACAGATGGAGAGGAATTTCACCC-AGAATGAATCTCACCCTCGCTTTCTCCCACACTT
γ3   CTATGCCTGCCCCATCATTGTACTTTGGAAGCAGATAACTTCTTGTCAAGTTACAAAGGTCCACAGATGGAGAGGAATTTCACCCCAGAATGAATC--ACCCTGCATTTCTCCCACACTT
γ4   ATGAGCAGATAGATGGGGGGGCAGTGGATCCCCAGGCACACACCCACACAGTGCACACAGCCCCACCTGGGCCAGAGGGGGGCAGGACGCCTCGCCACCCCTGCTGTGCTTTCTCCCACACTT
                                     BamHI                           *      *  * **                *            └─────→ σγ4

                                                                                                    ┌─────────┐
μ-δ  GATGCACGTGATATTTCGCTGAGATTGTGGACTAAGAGTTGGTGCTGGAAGGGGTCAGCCGTTCTGGAGATGTTGCTATGGGATGCACGGATTTTGCATGTGAGAACGACATGATTATCG
γ3   GATGCACGTGATATGTCGCTGAGATTGTGGACTAAGAGTTGGTGCTGGAAGGGGTTAGCCATCGTGGAGATGTTGCTATGGGATGCACGGATTTTGCCTGTCAGAACGACATGATTATCG
γ4   GATGCACGTGATATTTCTCTGAGATTGTGGACTAAGAGTTGGTGCTGGAAGGGGTTAGCCATCTTGGAGATGTTGCTATGGGGTGCACGGATTTTGCATGTGAGAACGACATGATTATCG
     **                                                              *        *         *                    *

                                                    ┌─────────┐
μ-δ  GGGGAACGGACGGCAAACTGTCATGGGTTAAAATGTGTCCCCTGTAAATAATGTGTCGAAGTCCTAACCCCCAGGACCACAGAATGTGACCTTGTCTGGAAACAGTCTTTGCAGCTGCA
γ3   CGGCAGCGGACGGCAAACTGTCATGGGTTAAAATGTGTCCCCTATAAATCATGTGTTGAAGTCCTAACCCCCAGGACCACAGAATGTGACCTTGTTTGGAAACAGTCTTTGCAACTGCA
γ4   CGGCAGTGGACGGCAAACTGTCGTGGGTTAAAATGTGTCCCCTATAAATCATGTGTTGAAGTCCTAACCCCCAGGACCACAGAATGTGACCTTGTTTGGAAACAGTCTTTGCAGCTGCA
              *                                                        *            *         *            *          *

μ-δ  ATCAAGTTCAGATGACGTCACCCTGGAGTAGGGCAAGCCTCTGATCCAATATGACTGCTGTCCTCATGAAAAGGGGGAATCTGGGCACAGACAGCACGTGGGGAGAACGCCCTGTGAACGA
γ3   ATCAAGTTCGGATGACGTCACCCTGGAGTAGGGCAAGCCTCTGATCCAATATGACTGCTGTCCTCATGAAAAGGGGGAATCTGGGCACAGAC-GCACGTGTGGAGAACGCCCTGTGAACGA
γ4   ATCAAGTTCAGATGACGTCACCCTGGAGTAGGGCAAGCCTCTGATCCAATATGACTGCTGTCCTCATGAAAAGGGGGAATCTGGGTACAGACAGCACGTGGGGAGAACACCCTGTGAACGA
                *                                                              *                * ** *    *      * *

                                                                                                          ┌──┐
μ-δ  TGGTGCTGCTTCCATAAGCCAAGAGCACCAGAGCCGGCCGGCAAAGCCCAGCAGCAGGGAGAGAGCCTGGAACAGAGTCTCCCGTGACACAGAGGAGCCAGCCCCGCCAAGGCCTCCATC
γ3   TGGTGCTGCTTCCACAAGCCAAGAGCAGCAGAGACGGCCGGCAAAGCCCAGCAGCAGCAAGGAGAGAGCCTGGAACAGAGTCTCC-ATGACACAGAGGAGCCAGCCCCACCGAGACCTCCATC
γ4   TGGTGCTGCTTCCATAAGCCAAGAGCAGCAGAGACGGCCGGCAAAGCCCAGCAGCAAGGAGAGAGCCTGGGACAGAGTCTCCCATGACACGGAGGTGCCAGCCCCGCCGACGCCTCCATC
                                                                                         * *

     ┌─────────────────────────────────────────────────────────────────────────────┐
μ-δ  CCAGATGCCCGGCCTCCAGAACCAGGACGGAATAAACGTCTGTTGTTTAAGGCACGCAGTCTGGGGTGCAGTGTTGCCAGGGCCCACAGTTAACGGATACGAGTGTTGTCCTGAGCTGCCA
γ3   CCAGATGACCGGCCTCCAGAACCAGGACGGAATAAACGTCTGTTGTTTAAGCCACGCAGTCTGGGGTGCAGTGTTGCCAGGGCCGCACTTAACGGATACGAGTGTTGTCCTGAGCTGCCA
γ4   CCAGATGCCCGGCCTCCAGAACCAGGACGGAATAAACGTCTGTTGTTTAAGCCACGCAGTCTGGGGTGCTGTGTTGCCAGGGCCACAGTTAACGGATACGAGTGTTGTCCTGAGCTGCCA
              *        *                                     *            *     *          * *

μ-δ  GCCCCACAGGCTGCACGAGGCCTCCCTGCCCCAGCCCAGTGCAGACTCCCCAGCCCCCTGGGTGTGCCCTGGGCAGTGTGGGGCTCCTCACTCCATCCTCCCCCAGGCTGGGGAGGTTGAG
γ3   GCCCCACAGACTGCACAAGGCCTCCCTGCCCCAGCCCAAGTGCAGTCTCCCCAGCCCCCTGGGTGTGCCCATGGGCAGTGTGGGGCCCATCACTCCGTCCTCCCCCAGGCTGGGGAGGTTGAG
γ4   GCCCCACAGCTGCACGAGGCCTCCCTGCCCCAGCCCAGTGCAGACTCCCCAGCCCCCTGGGTGTGCCATGGGCAGTGCGGGGCCCTCACTGCATCCTCCCCCAGCCTGGGGAGGTTGAG
              * **    * *       *       *                              *

μ-δ  CCTGTGATGAGCTACATGGGGTGAAGCTGGAGCGAGAGGCTGGGAGGCGACTCGGAGCCCAGGTTGGAGGATGGATTTCCCCAGGGACCCACACGTGCACCTCCACCTGTCTCCTGGAC
γ3   CCTGTGATGAGCTCCATGGGGTGAAGCTGGAGCGAGAGGCTGGGAGCCGACTGGGAGCCCGCGGCTGGAGGATGGATTTCCCCAGGGACCCACACGTACACCTCCACCTGTCTCCTGGAC
γ4   CCCATTATGAGCTCCATGGGGTGAAGCCGGAGCCAGAAGCTGGGAGCCGACTGGG--CCTGCGGCTGGAGGATGGATTTCCCCAGGGACCCACACGTGCACCTCCACCTGTCTCCTGGAC
     **  *        *              *       *                       ** **                *

μ-δ  ATTGTCTCTGACGGCAGGGCTGGTGCCAGCTCAGGGATCCAGCAGGGACAGAAGGGCGGGGCCGGGTCCATGTGGAGAGCACATTTAGTGGGAGGGACACAACTTGTACCCAGCAGCCCG
γ3   GTTCTCTCTGACGGCAGGGCTGGTGCCAGCTCAGGGATCCAGCAGGGACAGAAGGGCGGGGCCGGGTCCTTGTGGAGAGCACATTTAGTGGGAGGGACATGATTTCCCCTCACAAGTCTCC
γ4   ATTCTCTCTGACGGCAGGGCTGGTGTCAGCTCAGGGATCCACCAGGGACACAAGGGTGGGGCCGGGTCCTTGTGGAGAGCACATTTAGTGGGAGGGACATGATTTCCCTTCA-AAGTGTCC
                                                                                              BamHI                    σγ ←────
```

(c)

EP 0 344 319 A1

```
                                      TG        CG
                                 CTGGA  CTTCCTGTTCCA    consensus sequence
                                      CA        TT

              *    *
      TGGGAGGGACATGATTTCCCCTCACAAGTGTCCATT    --------CTTCCTGTTCCTTG
γ3                                           CTGGACGCTTCCTCTTCCATT           ******  ***      **    **  .*  .*
        σγ3                                  CTGGACACTTCCTGTGCGACA    CCTCCTCGGGCTTT--------CCCGAGCCCTCTGGCCTCATTCCGTTCCCTCCTACC

      TGGGAGGGACATGATTTCCCTTCA-AAGTGTCCATT   CTGGATGCTTCCTGTTCCACG
γ4                                           CTGGACACTTCCTGTTCCACG
        σγ4                                  CTGGACGCTTCCTGTTCCACG
                                             CTTGACGCTTCCTGTTCCACG
                                             CTTGACGCTTCCTGTTCCATG
                                             CTGGATGCTTCCTGTTCCATG
                                             CTGGACATTTCCTGTTCCACT
                                             CTGGATGCTCCCTGTTCCATT
                                             CTGGATGCTTCCTGTTCCATG
                                             CTGGACATTTCCTGTTCCACT
                                             CTGCATGCTTCCTGTTCCACT
                                             CTGGATGCTTCCTGTGCGAAA   CCTCCTCGGGCTTTTGGTCTGCCCAGTCCCTCTGGCTGCATCTCGTCCCCCGCTACC
         *        * *                           *            *    **                                               *      *
γ3   TCCCACTTCCACGTACGTCCTTGCCCAGCTCTTCCCTCTATCCAGAGCTTCTGCCTGGCAAGGTCCCTGCTGAGATCAGTCCAGGCTCCCCCAGCACAGGTAGGAGCCTTGCACATCCCC
γ4   TCCCACCTCCACATCCGTCCTTGCCCAGCTCCTCTCTCTCTCCAGAGTTTCCACCTGGCAAGGTCCCTGATGAGCTCAGTCCAGGCTCCCCCAGCACAGGTAGGAGCCTAGCACCTGCCC
                                                                          *    *  *  *                       ** *            *
γ3   TTGGACCTCCCCACCCTGCATGATGCCAGCATCCCCAGGCCCCAGGGAGGCCCCATTTCTCTCTCTGCTGGTAGTCCAGTGGCCCTGGAGTCCCACTGCAGGTGCGGGTGTGCCCCTGAAC
γ4   TTGGACCTCCCCACCCTGCATGATGCCAGCATCCCCAGGCCCCAGGGAGGCCCCATTTCTCTCTCTACTGCTGGCCCAGTGGCCCTGGAGTCCCACTGCAACTCCGGTGTGCCCCTGACC
        *         **     *   **  *                    *                          *                        ** .          *
γ3   TCTGACGAAGCTAAGTACCCTGCCCTCAGACACGCTATCCCCCCTGCTCAGCCCCAGGGCCCTGCCCCCTACCCCTTCCCCTCACCTGCACCACAGGCTCTGGCCCAACTCTTCCCACGCC
γ4   TCTGACGAAGTTAAGTGTCCTGTCCCTAGCCAGGCTATCCCCTGTGCTCAGCCCCAGGGCCCTGCCCCTTACCCCTTCCCCTCACCTGCACGATAGGCTCTGGCCAACTCTGCCCACGCC
                                                                                                     *
γ3   CTGAATGGGCCCCTCTGGCTCCCCTCTGCTGCTACACTGCCCTGCACCACCTCCACTCAGCTTCAGTGTGTTCATCCGCCTGTCCCACGTCCCCTCGGCCCCCAGGAGCACAGCTGGTCG
γ4   CTGAATGGGCCCCTCTGGCTCCCCTCTGCTGCTACACTGCCCTGCACCACCTCCACTCAGCTTCAGTGTGTTCATCCACCTGTCCCACGTCCCCTCGGCCCCCAGGAGCACAGCTGGTCG
        *                                                                                                      *
γ3   CCCTCGCTCCTCGCAGCCCATCTTGTTCCTTCTGGAGCACCAGCCTCAGAGGCCTTCCTGTGCAGGGTCCACTCGGCCAGCCCTGGGACCCTCCTGGTCTCAAGCACACACATTCTGCCT
γ4   CCCTCGTTCCTGGCAGCCCATCTTGTTCCTTCTGGAGCACCAGCCTCAGAGGCCTTCCTGTGCAGGGTCCACTCGGCCAGCCCTGGGACCCTCCTGGTCTCAAGCACACACGTTCTCCCT
                                                       *
γ3   GCAGCCAGACCTGCCCCTGCCTGTGAGTTCAGACCTGAGCCTTGGAACGCCTTCCCTTCTCCATCCCAGCTCGCCTTTGCCAGCTGCTCAGCAGGATGAACTCACACTCCCCTCCCTGCA
γ4   GCAGCCAGACCTGCCCCTGCCTGTGAGTTCAGACCTGAGCCTTGGAACGTCTTCCCTTCTCCATCCCAGCTCGCCTTTGCCAGCTGCTCAGTGGGATGAACTCACACTCCCCTCCGTCCA
               *                                           *
γ3   CCATGAGTGAGAGTCAGCTGGAGAGATGCCCAGGCAAAAGCAGCCACCAGGGCCCAGTGGGGG-CCAGAAGCTT
γ4   CCATGAGTGAGAGTCAGCTGGAGAGATGCCCAGGCCAAAGCAGCCACCAGGGCCCAGTGGGGGGGCCAGAAGCTT
                                                                      HindIII
```

Fig. 9

EP 0 344 319 A1

## (a)

```
        1      1000   2000   3000   4000   5000   6000   7000   8000
        |       |      |      |      |      |      |      |      |
        |       |      |      |      |      |      |      |      |
```

human

Cμ   Z-DNA        μm  49bp 35bp   Σ₁        Σμ   63bp        Cδ

Σ

mouse

Cμ   Z-DNA   μm        76bp    Orf-146   Cδ

## (b)

human  σ₁₃   ACTGCCGTCGGC----TTAAACAACAGACGTTTATTCCGTCCTCGGTTCTGGAGGCCCGGTCATCTCGGAT

σ₁₄   ACTGCCGTCGGC----TTAAACAACAGACGTTTATTCCGTCCTCGGTTCTGGAGGCCCGGGCTACTGCGAT

Σ

μ-δ   ACTGCCGTCGGC----TTAAACAACAGACGTTTATTCCGTCCTCGGTTCTGGAGGCCCGGGCATCTCGGAT

μ-δ   ACTCCATGCC----TGAAACAACAGAAGTGTATTCTGTGTCATTTCTCGGAGGCCCAGGTGTCTCAGAT

mouse  μ-δ   5'CACACATGGCAGTTTTCAGGCAGGCAGAACTGTGTTCTTTTCCTAGTTCTAGAGGCCACGAAGTGTGGGCC----

|||  ||||||||||||||||||  ||||||  |||||||||||||||||  ||||||||||||  ||  |||  ||||||

3'GTCGATACCGTCAAACTCGTTGTCTTAAGACAAGAAAGGGACAAGATCTCCGATCCTCAGACCCGA----

Fig. 10

Fig. 11

(a)

$C_{l2b}$   $C_{l2a}$

MEP 12

| EcoRI
| Hind III
▼ BamHI

1 kb

(b)
CCATCCATACCCTGAGATACCACACACACGCTCAGAAAAATGCTTCTCGAATGTACGAGTGTTACCATCAATGGAGACGTAACGTACACAATCTAACCCC
BamHI
TCCTAACCAAACACCTAAAACACGACCCACGTGCTACCTCCCCAGTGAGTTCTCAGATCGAATTTACATGTCTTAAGATATATCCTTGACTGTTAGAGAC
TGTTCAAGTCTATATTCTCTCTAGTCGTACCTAATTGAGAAAACTCAACGCCCCTCGAACACGTCAAGAAAAGATCCTGTCACCACAGCTACAGAGAGAG
CAACACCCCGCAGATTACTAACTGGTCAGTACTATCACAGCCTCTGGTGTTCGGAAACGCATGCTCTACCTAGAACTGAGGAGCACCAGATTCCTGTGAG
TGGTATTCCTCCTCGACAACCCATAGGGAGATGGACAGTGGAGGTATTCATAGCTCATGCACAGACAGCTGCATGCAGCCTGGACTTTAGTCAAGAGTAC
TCACCACCTACTACAAAACTGCGAACTTACGACACACAGCTGGGAGTAAGAACAGTGGACCCTAGACTACAAAGACGACAGCTGCCCTAACCCTGAGAAA
AGCTATTTAATCGTCGACAAACCTCTCGTCACTGTCCAACCAAACATCCCTGTCCAAAGCAGTGAAGCAACGACACAGATCATCAGTTTAGAGATCATTT
AGCGCTCCCACTGTTTTTCTTCCTCTGCATAACACGCAAATGTTCGGTTTGTGTCAGAGTTGAAGTGTACAGTCCGGCACAGCCAAGCAGTCTTCGCTTTGTTTC
GCTCCCAGTGCATAAAGTATCCTGCACAATAGCCAGTTTTCCTCAATCGCCAGTTTAAGCTT
HindIII

(c)

Fig. 12

EP 0 344 319 A1

(a)

(b)

(c)  (d)  (e)

Fig. 13

Fig. 14

Fig. 15

EP 0 344 319 A1

# INTERNATIONAL SEARCH REPORT

**I. CLASSIFICATION OF SUBJECT MATTER** (if several classification symbols apply, indicate all) [6]

According to International Patent Classification (IPC) or to both National Classification and IPC

Int.Cl[4]    C12N15/00, C12P21/00

**II. FIELDS SEARCHED**

Minimum Documentation Searched [7]

| Classification System | Classification Symbols |
|---|---|
| IPC | C12N15/00, C12P21/00-21/02 . |

Documentation Searched other than Minimum Documentation
to the Extent that such Documents are Included in the Fields Searched [8]

Biological Abstracts Data Base (BIOSIS)

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| X | Nucleic Acids Research, Vol. 12, (1984) Celia P. Milsteinetal. [The sequence of the human immunoglobulin μ-δ intron reveals possible vestigial switch segments] p.6523-6535 | 3-4, 15-19 |
| A | Nucleic Acids Research, Vol. 12, (1984) Celia P. Milstein et al. [The sequence of the human immunoglobulin μ-δ intron reveals possible vestigial switch segments] p.6523-6535 | 1-2, 5-14 20, 25-30 |
| A | Nature, Vol.300, (1982) J.G. Flaragan et al. [Arrangement of human immunoglobulin heavy chain constant region genes implies evolutionary duplication of a segment containing γ, ε and α genes] p. 709-713 | 5-18, 20-26 |
| A | Nature, Vol.302, (1983) Susumu Tonegawa [Somatic generation of antibody diversity] p.575-581 | 1-30 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 5, 1989 (05. 01. 89) | January 17, 1989 (17. 01. 89) |
| International Searching Authority | Signature of Authorized Officer |
| Japanese Patent Office | |